# EUROPEAN PATENT SPECIFICATION

(11) **EP 3 977 848 B1**
(45) Date of publication and mention of the grant of the patent: **25.09.2024**
(21) Application number: 20382869.4
(22) Date of filing: 30.09.2020
(51) Int. Cl.: A01K 67/0275, A01K 67/0276, C07K 14/47, G01N 33/50

(54) **TRANSGENIC MOUSE MODEL OF MYELIN PATHOLOGIES**
TRANSGENES MAUSMODELL VON MYELINPATHOLOGIEN
MODÈLE DE SOURIS TRANSGÉNIQUE DE PATHOLOGIES MYÉLINIQUES

(43) Date of publication of application: 06.04.2022
(73) Proprietor: Universidad Autónoma de Madrid, 28049 Madrid (ES)
(72) Inventor: CUBELOS ÁLVAREZ, Beatriz, 28049 Madrid (ES); ALCOVER SÁNCHEZ, Berta, 28049 Madrid (ES)
(74) Representative: Hoffmann Eitle

(56) References cited:
- US-A1- 2006 282 905
- MIRIAM SANZ-RODRIGUEZ ET AL: "R-Ras1 and R-Ras2 Are Essential for Oligodendrocyte Differentiation and Survival for Correct Myelination in the Central Nervous System", THE JOURNAL OF NEUROSCIENCE, vol. 38, no. 22, 30 May 2018 (2018-05-30), US, pages 5096 - 5110, XP055726066, ISSN: 0270-6474, DOI: 10.1523/JNEUROSCI.3364-17.2018
- ALCOVER-SANCHEZ BERTA ET AL: "R-Ras GTPases Signaling Role in Myelin Neurodegenerative Diseases", INTERNATIONAL JOURNAL OF MOLECULAR SCIENCES, vol. 21, no. 16, 17 August 2020 (2020-08-17), pages 5911, XP055778891, Retrieved from the Internet <URL:https://www.ncbi.nlm.nih.gov/pmc/articles/PMC7460555/pdf/ijms-21-05911.pdf> [retrieved on 20210223], DOI: 10.3390/ijms21165911

## Description

### FIELD OF THE INVENTION

The present invention refers to the medical field. Particularly, it refers to a transgenic mouse model of myelin pathologies (or transgenic oligodendrocytes and/or neurons derived thereof). Moreover, the present invention also refers to the use of the mouse model of the invention (or oligodendrocytes and/or neurons derived thereof) in a method for screening candidate compounds for treating myelin pathologies.

### STATE OF THE ART

Myelin is critically required in the vertebrate nervous system to enable fast and efficient synaptic transmission. In the central nervous system (CNS), oligodendrocytes (OLs) create myelin and extend processes that wrap around axons and form compact myelin sheaths. These multilayered membrane compartments, or nodes, line up along the axon to provide metabolic support and protection from the extracellular space. Between nodal compartments lies Nodes of Ranvier, structures in which the molecular machinery responsible for electric transmission is concentrated. This organization enables fast saltatory conduction of action potentials initiated from the neuronal soma because axonal membrane depolarization skips from node to node, a feature believed to contribute to vertebrate evolution.

Myelin's role is so important that when it is impaired or lost, pathologies begin to appear. The onset of myelin dysfunction can impact a wide range of processes from embryonic development to chronic degenerative diseases such as multiple sclerosis. At a cellular level, loss of myelin causes channels responsible for depolarization to become distributed along the axon instead of being concentrated in Nodes of Ranvier, requiring greater number of depolarizations and increased use of ATP to fuel synaptic transmission. When whole-cell ATP levels are depleted, Na⁺/K⁺ ATPase is then unable to restore the Na⁺/K⁺ gradient after an action potential, which leads to degeneration and axonal dysfunction. To compensate for increased axonal energy demands, both numbers and activity of mitochondria in demyelinated areas increase, a well characterized feature of demyelinating diseases demonstrated both in humans and in numerous animal models.

Among the pathologies associated with myelin, sensorial, motor and cognitive alterations stand out. Some of the most important pathologies that affect the CNS are: multiple sclerosis (MS), neuromyelitis optica (NMO), hypomyelinating leukodystrophies and Charcot-Marie-Tooth disease (CMT). For example, in the case of Multiple Sclerosis alone, 1,800 new cases are diagnosed each year in Spain alone. Multiple sclerosis affects 47,000 people in Spain, 700,000 people in Europe and 2.5 million people worldwide. In the past two decades, the number of patients with multiple sclerosis has doubled. The average delay in the diagnosis and treatment of multiple sclerosis is between one and two years. Multiple sclerosis is the second cause of disability among Spanish youth, after traffic accidents.

Multiple sclerosis is the most prevalent CNS neurological disease that affects young adults. In fact, it is generally diagnosed at the age of 20-40 years and is approximately 2 or 3 times more frequent in women than in men. The etiology remains unknown; however, it is believed to occur as a result of some combination of genetic and environmental factors. Today, MS is considered a chronic, autoimmune, and neurodegenerative disease that affects more than 2 million people worldwide. This neurodegenerative disease is characterized by demyelination with concomitant axonal and neuronal degeneration that causes a heterogeneous variety of symptoms and signs.

Another neurodegenerative disease is neuromyelitis optica (NMO, previously called Devic's disease). It is considered a rare, autoimmune, demyelinating CNS disease that has optic neuritis and manifestations of acute transverse myelitis. This disease affects more than 2 million people worldwide and has a prevalence of 1-3 per 100,000 and is more common in women than in men (from 2: 1 to 10: 1). It was first considered as a monophasic syndrome that was a subtype of MS until the discovery of autoantibodies against aquaporin-4 (AQP4-IgG). Furthermore, among patients with NMO there is a seronegativity of 10% to 25% for AQP4-IgG, which suggests the participation of other factors in the development of the disease, such as autoantibodies against myelin oligodendrocytic glycoprotein (MOG-IgG).

On the other hand, leukodystrophies are rare diseases, often genetic, characterized by a loss of myelin in the CNS and PNS. They have very diverse origins, such as mutations that affect different cellular compartments, cellular processes, or protein translation. Only some genes whose mutations cause leukodystrophies encode myelin diseases, such as Pelizaeus-Merzbacher disease (PMD) where the PLP1 gene is mutated.

Another neurodegenerative disease is Charcot Marie-Tooth disease classified as sensory and motor neuropathy. It is a phenotypic and genetically heterogeneous group of classically primary genetic neuropathies. CMT is the most common hereditary neuropathy worldwide with a prevalence of 1: 2,500, and it is a childhood-onset syndrome. It has enormous heterogeneity due to the different inheritance patterns (autosomal dominant, autosomal recessive, X-linked) classified into different electrophysiological classes characterized by electromyography (axonal, demyelinating, intermediate). The most frequent pattern is autosomal dominant inheritance.

Despite the advances in the study of myelin diseases (for instance amyotrophic lateral sclerosis, neuromyelitis optica, multiple sclerosis, Charcot Marie Tooth disease or leukodystrophies), it is still needed a greater insight from a neurological perspective. Nevertheless, in order to gain insight into these human diseases it is important to generate animal models which faithfully reproduce the human condition. This would give the researchers the possibility of better understanding these pathological conditions and assaying effective treatments.

Currently there are two animal models for the study of myelin diseases. Both models are based on the artificial aggression of the animal's immune system in order to reproduce the immune symptoms:
- Immune (EAE): An autoimmune reaction is caused by direct immunization after injection of myelin proteins. This generates an immune response of the animal against these injected foreign antigens. This system is the Experimental Autoimmune Encephalomyelitis (EAE) model. In this model, the animal's immune system produces antibodies that attack and destroy myelin.
- Viral: This model is an inoculum of coronavirus (murine hepatitis virus), enterovirus (Theiler virus), lentivirus or canine distemper virus. The most widespread is the model based on inoculation of Theiler virus that produces murine demyelinating encephalomyelitis caused by Theiler virus (TMEV-IDD Theiler murine encephalomyelitis virus-induced demyelinated disease). These models are included in the autoimmune reaction caused by the injection of viruses that in an unknown way presented an immune response of the animal against the myelin antigens due to the similarity it possesses with the virus's own antigens.

Currently, these models are used to validate the therapeutic effect of new compounds. However, although some compounds have been effective in these models, this effect was not been reproduced in preclinical trials (including aggravated symptoms such as IFN-gamma). Therefore, the development and improvement of animal models which faithfully reproduce the pathophysiology of these diseases is of utmost importance. New models that reproduce the symptomatology of these diseases from a neurological point of view is therefore a priority of crucial importance because this would allow the study of the intrinsic factors that can elucidate possible treatments for these diseases.

In this sense, prior art documents [Miriam Sanz Rodriguez, et al. 2018. R-Ras1 and R-Ras2 Are Essential for Oligodendrocyte Differentiation and Survival for Correct Myelination in the Central Nervous System. J Neurosci. 2018 May 30;38(22):5096-5110. doi: 10.1523/JNEUROSCI.3364-17.2018. Epub 2018 May 2] and WO2017/055930 disclose single and double knockout mice characterized by the disruption or inactivation of R-Ras1 and/or R-Ras2. However, the loss of function of R-Ras1 and/or R-Ras2-/-, is not enough for the constitution of a model for the study of myelin diseases from a neurological perspective. Kindly note that myelin pathologies have a multifactorial etiology, and there is evidence of a great neurological contribution in their development. However, current animal models for these diseases are exclusively based on reproducing immune system abnormalities associated with these diseases. In other words, there is a need of new animal models that reproduce neurological symptoms, which may be used for screening or assaying new drugs for treating myelin pathologies from a neurological point of view. Please note that Miriam Sanz-Rodriguez, et al. 2018 and WO2017/055930 do not disclose animal models with the required phenotypic features to be a reliable animal model which reproduces neurological symptoms of the myelin pathologies.

The present invention is thus focused on solving this problem and it is herein proposed a transgenic mouse model of myelin pathologies that reproduces the symptomatology of these diseases from a neurological point of view and faithfully resembles the human condition. The animal model of the invention, apart from showing a disruption or inactivation of the genes R-Ras1 and/or R-Ras2, is characterized by several phenotypic features that reproduce the neurological burden associated with myelin pathologies such as: i) increased amount of immature oligodendrocytes, ii) decrease amount of mature oligodendrocytes iii) presence of demyelinated axons iv) several mitochondrial adaptations, v) several metabolism alterations in myelin deficient tissues, vi) axonal degeneration and vii) functional alterations. Again, kindly note that Miriam Sanz-Rodriguez, et al. 2018 and WO2017/055930 do not disclose animal models comprising all those phenotypic features that reproduce the neurological burden associated with myelin pathologies.

### DESCRIPTION OF THE INVENTION

### Brief description of the invention

Myelin pathologies have a multifactorial etiology, and there is evidence of a great neurological contribution in their development. Thus, it is herein proposed a paradigm switch in the study of myelin diseases from a new point of view, focusing on a neurological model.

Consequently, the inventors of the present invention have developed a new model that faithfully reproduces the symptomatic characteristics of myelin pathologies from a neurological point of view. This is highly relevant because myelin pathologies are in fact neurodegenerative diseases. What is even more relevant is that the animal models of the present invention do not have a functional immune system, so this component would not be decisive in finding a treatment. This means that the capacity of drugs to myelinate could be really evaluated in physiological conditions very similar to those presented in these diseases.

According to the inventors of the present invention, in order to be a good animal model for the study of myelin diseases from a neurological point of view, the animal model should have some phenotypic features which resembles the neurological burden of the myelin pathologies. Thus, the animal model of the invention is characterized by several phenotypic features that reproduce the neurological burden associated with myelin pathologies such as: i) increased amount of immature oligodendrocytes, ii) decreased amount of mature oligodendrocytes iii) presence of demyelinated axons iv) several mitochondrial adaptations, v) several metabolism alterations in myelin deficient tissues, vi) axonal degeneration and vii) functional alterations.

Transgenic knock-out *R-Ras2*^{*-*/*-*} or double knock-out *R-Ras1*^{*-*/*-*} and *R-Ras2*^{*-*/*-*} mouse model of myelin pathologies is characterized by the following phenotypic features:
a) A 70% to 155% increase of immature oligodendrocytes in the double knock-out R-*Ras1*^{*-*/}*⁻R-Ras2*^{*-*/*-*} transgenic mouse model, with respect to the number of immature oligodendrocytes measured in healthy controls, determined by immunostaining with specific antibodies against Tcf4 and O4 and quantified by fluorescence microscopy;
b) At least 50% decrease of mature oligodendrocytes in the double knock-out *R-Ras1*^{*-*/}*⁻R-Ras2*^{*-*/*-*} transgenic mouse model, with respect to the number of mature oligodendrocytes measured in healthy controls, determined by immunostaining with specific antibodies against Olig2 and CC1 and quantified by fluorescence microscopy;
c) At least 20% of demyelinated axons in the double knock-out *R-Ras1*^{*-*/}*⁻R-Ras2*^{*-*/*-*}transgenic mouse, and at least 70% of demyelinated axons in the knock-out *R-Ras2*^{*-*/*-*}as compared with heathy controls, determined by electron microscopy;
d) Mitochondrial adaptations comprising:
   a. Increased mitochondrial number per non-myelinated axon of 0.61 ± 0.18 for the knock-out *R-Ras2*^{*-*/*-*} and 0.75 ± 0.23 for the double knock-out *R-Ras*^{*1-*/}*⁻R-Ras2*^{*-*/*-*} as compared with the number of 0.22 ± 0.08 measured in healthy control, determined by electron microscopy;
   b. Decreased average mitochondrial area in non-myelinated axons of 0.091 ± 0.05 mm² for the knock-out *R-Ras2*^{*-*/*-*} and 0.083 ± 0.03 mm² for the double knock-out *R-Ras1*^{*-*/}*⁻R-Ras2*^{*-*/*-*} as compared with the number 0.14 ± 0.08 mm² measured in healthy control, determined by electron microscopy;
   c. Increased protein expression of the following respiratory chain elements in the double knock-out *R-Ras1*^{*-*/}*⁻R-Ras2*^{*-*/*-*}: ATPsyn fold change of 2.08 ± 0.84, COXIV fold change 2.67 ± 0.21 and UQCRC2 fold change 3.40 ± 0.92, relative to the expression measured in healthy control, determined by western-blot;
   d. ADP-stimulated State III in the double knock-out *R-Ras1*^{*-*/}*⁻R-Ras2*^{*-*/*-*} of 31.3 ± 3.1 nmol O/ min / mg protein relative to healthy control, determined with Clark electrode;
e) Metabolism alteration in myelin deficient tissues comprising:
   a. Lactate/pyruvate ratio of 121.75 ± 14.44 for the knock-out R-Ras2^{-/-} and 116.19 ± 16.42 for the double knock-out *R-Ras1*^{-/-}*R-Ras2*^{-/-} as compared with the number 153.53 ± 18.11 measured in healthy control, determined by gas chromatography-mass spectrometry;
   b. Pyruvate amount of 0.027 ± 0.003 for the knock-out R-Ras2^{-/-} and 0.024 ± 0.003 for the double knock-out *R-Ras1*^{-/-}*R-Ras2*^{*-*/*-*} as compared with the number 0.022 ± 0.004 measured in healthy control, determined by gas chromatography-mass spectrometry;
   c. Lactate amount of 3.314 ± 0.405 for the knock-out R-Ras2^{-/-} and 2.918 ± 0.720 for the double knock-out *R-Ras1*^{*-*/}*⁻R-Ras2*^{*-*/*-*} as compared with the number 3.519 ± 0.640 measured in healthy control, determined by gas chromatography-mass spectrometry;
   d. Citrate amount of 0.021 ± 0.004 for the knock-out R-Ras2^{-/-} and 0.028 ± 0.010 for the double knock-out *R-Ras1*^{*-*/}*⁻R-Ras2*^{*-*/*-*} as compared with the number 0.028 ± 0.010 measured in healthy control, determined by gas chromatography-mass spectrometry;
   e. Succinate amount of 0.028 ± 0.004for the knock-out R-Ras2^{-/-} and 0.021 ± 0.007 for the double knock-out *R-Ras1*^{*-*/}*⁻R-Ras2*^{*-*/*-*} as compared with the number 0.029 ± 0.007 measured in healthy control, determined by gas chromatography-mass spectrometry;
   f. Glycine amount of 0.139 ± 0.057 for the knock-out R-Ras2^{-/-} and 0.131 ± 0.040 for the double knock-out *R-Ras1^{-l-}R-Ras2*^{-/-} as compared with the number 0.146 ± 0.031 measured in healthy control, determined by gas chromatography-mass spectrometry;
   g. Lysine amount of 0.003 ± 0.001 for the knock-out R-Ras2^{-/-} and 0.004 ± 0.001 for the double knock-out *R-Ras1*^{*-*/}*⁻R-Ras2*^{*-*/*-*} as compared with the number 0.007 ± 0.002 measured in healthy control, determined by gas chromatography-mass spectrometry;
   h. Proline amount of 0.035 ± 0.011 for the knock-out R-Ras2^{-/-} and 0.040 ± 0.013 for the double knock-out *R-Ras1*^{*-*/}*⁻R-Ras2*^{*-*/*-*} as compared with the number 0.047 ± 0.012 measured in healthy control, determined by gas chromatography-mass spectrometry;
f) Axonal degeneration determined by the following parameters:
   a. ROS increment for the double knock-out R-Ras1^{-/-}R-Ras2^{-/-} of 162.4 ± 53.4% compared to healthy control, determined by western-blot;
   b. APP increment for the double knock-out *R-Ras1*^{*-*/}*⁻R-Ras2*^{*-*/*-*} of 154.52 ± 18.71% relative to healthy control, determined by western-blot and confirmed by immunostaining and confocal microscopy;
   c. Astrogliosis for the double knock-out *R-Ras1*^{*-*/}*⁻R-Ras2*^{*-*/*-*} of 173.95 ± 24.71% relative to healthy control, determined by immunostaining with antibodies against GFAP and confocal microscopy;
   d. Microgliosis for the double knock-out *R-Ras1*^{*-*/}*⁻R-Ras2*^{*-*/*-*} of 131.75 ± 16.08% relative to healthy control, determined by immunostaining with antibodies against Iba1 and confocal microscopy;
   e. Alteration of the phosphorylation state of the element of the axonal cytoskeleton Tau1: 47.7 ± 22.1% for the knock-out R-Ras2^{-/-} and 37.4 ± 3.8% for the double knock-out *R-Ras1*^{*-*/}*⁻R-Ras2*^{*-*/*-*} relative to healthy control, determined by western-blot;
   f. Alteration of the phosphorylation state of the element of the axonal cytoskeleton SMI-32: 58.7 ± 25.5% for the double knock-out *R-Ras1*^{-/-}*R-Ras2*^{*-*/-} relative to healthy control, determined by immunostaining and confocal microscopy;
   g. Expression of PSA-NCAM 50.0 ± 12.9% for the double knock-out *R-Ras1*^{-/-}*R-Ras2*^{-/-}relative to healthy control, determined by immunostaining and confocal microscopy;
   h. Increase in apoptotic processes in ganglion cells of 169.02 ± 4.47% for the double knock-out R-Ras1^{-/-}R-Ras2^{-/-} relative to healthy control, determined by immunostaining and confocal microscopy;
g) Functional alterations comprising:
   a. Percentage of correct step sequences over the total number of step cycles of 51.66 ± 27.23 for the double knock-out R-Ras1^{-/-}R-Ras2^{-/-}, 61.67 ± 8.81 for the knock-out R-Ras2^{-/-} as compared to the percentage determined in healthy control 87.50 ± 6.66, determined by paw-print test;
   b. Stride length of 50.30 ± 5.08 mm for the double knock-out R-Ras1^{-/-}R-Ras2^{-/-} as compared to the healthy control 60.37 ± 3.93 mm, determined by paw-print test;
   c. Contrast sensitivity in the range of 0.011-0.355 cycles/degree was significantly weaker for the double knock-out R-Ras1^{-/-}R-Ras2^{-/-}, determined by optomotor test.

In a preferred embodiment, there is an absence of mitochondrial adaptations in purified cultures of neurons or purified cultures of oligodendrocytes. This indicates that the mitochondrial alterations actually occur when both cell types (neurons and oligodendrocytes) interact. In other words, the information obtained from purified cultures of oligodendrocytes or isolated neurons regarding the mitochondrial adaptations, is crucial for understanding the importance of the interactions between these cell types.

The transgenic knock-out mouse is characterized in that it is a model of a myelin pathology selected from the group comprising: amyotrophic lateral sclerosis, neuromyelitis optica, multiple sclerosis, Charcot Marie Tooth disease and leukodystrophies.

The present invention refers to an *in vitro* method for screening candidate compounds for treating myelin pathologies, which comprises: a) determining in the mouse model or the oligodendrocytes or neurons of the invention the level of expression of R-Ras1 and *R-Ras2,* and the parameters a) to g) described above after administering the candidate molecule and b) where, if after administering the candidate molecule, the parameters a) to g) described above are similar to the values determined in healthy control, this is indicative that the candidate molecule is effective in the treatment myelin pathologies.

In a preferred embodiment, the myelin pathology is selected from the group comprising: amyotrophic lateral sclerosis, neuromyelitis optica, multiple sclerosis, Charcot Marie Tooth disease and leukodystrophies.

The present invention also refers to an in vitro method for obtaining a transgenic mouse model of myelin pathologies which comprises the disruption or inactivation of the gene R-*Ras2,* or R-Ras1 and *R-Ras2,* and determining and establishing the parameters a) to g) described above.

For the purpose of the present invention the following terms are defined:
- The term "comprising" includes, but it is not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.

### Description of the figures

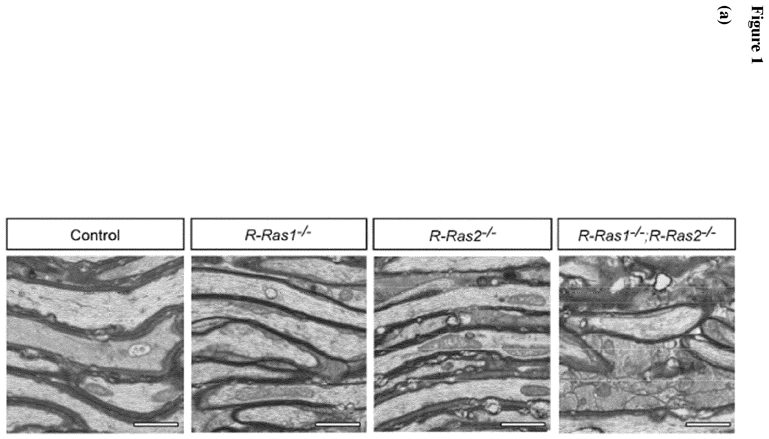

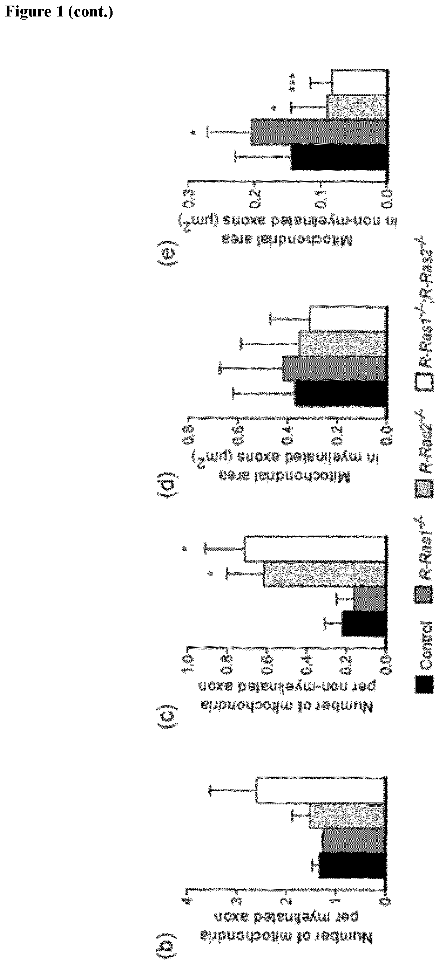

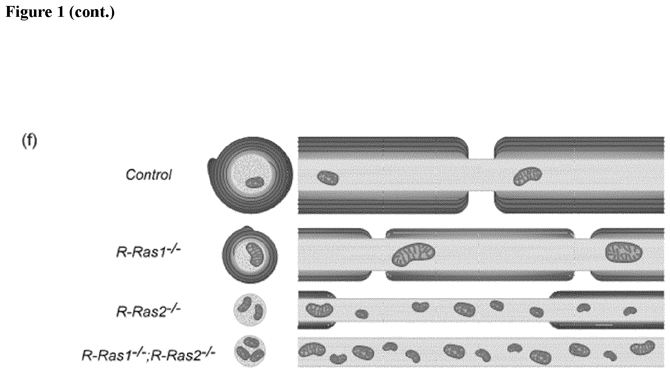

**Figure 1****.** Loss of R-Ras2 affects axonal myelination of the optic nerves causing an increase in the mitochondria in non-myelinated axons. (a) Electron microscopy of the longitudinal sections of the optic nerve of adult (P90) control, *R-Ras1*^{*-*/}*⁻, R-Ras2*^{*-*/*-*} and *R-Ras1*^{*-*/}*⁻; R-Ras2*^{*-*/*-*}double knockout mice. There was a significant increase in non-myelinated axons in the absence of R-Ras2 in single and double-mutants compared to controls. (b) The number of mitochondria per myelinated axon in the optic nerve of adult mice (P90) revealed no differences between single and double-mutants relative to controls. (c) The number of mitochondria per non-myelinated axon present in the optic nerve of adult mice (P90) showed significant differences between *R-Ras2*^{*-*/*-*} (*p<0.05) and the double-mutant mice (**p*<0.05). (d) The average number of mitochondria area per myelinated axon present in the optic nerve of adult mice (P90) revealed no differences between single and double-mutants relative to controls. (e) The average number of mitochondria area per non-myelinated axon present in the optic nerve of adult mice (P90) showed significant differences in *R-Ras1*^{*-*/*-*} (**p<0.05*), *R-Ras2*^{*-*/*-*} (*p<0.05), and the double-mutant mice (***p<0.001). (f) Schematic representation of the mitochondria alteration in the longitudinal and transverse sections of the axons with myelin deficits in singles and double mutant mice relative to controls. (n = 3 mice per genotype). Bar graph represents the mean ± SD of controls. Two-tailed Student t-test was used for the statistical analysis. Scale bar, 1 µm. SD, standard deviation.

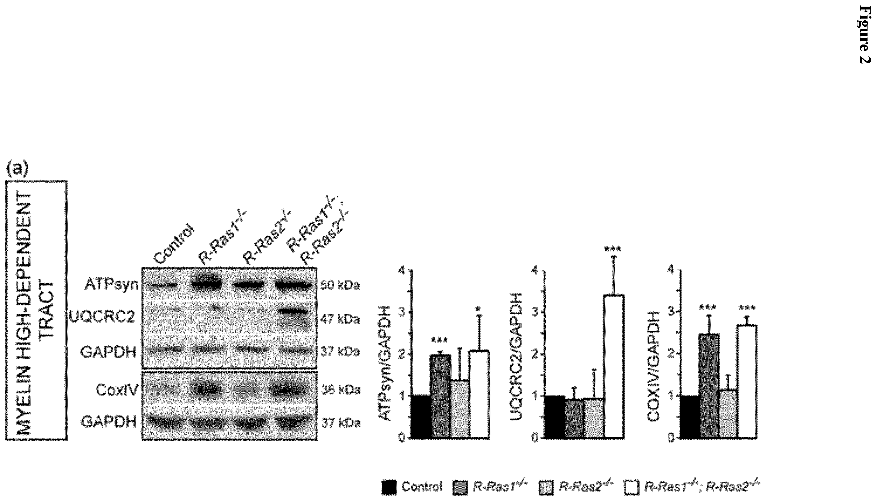

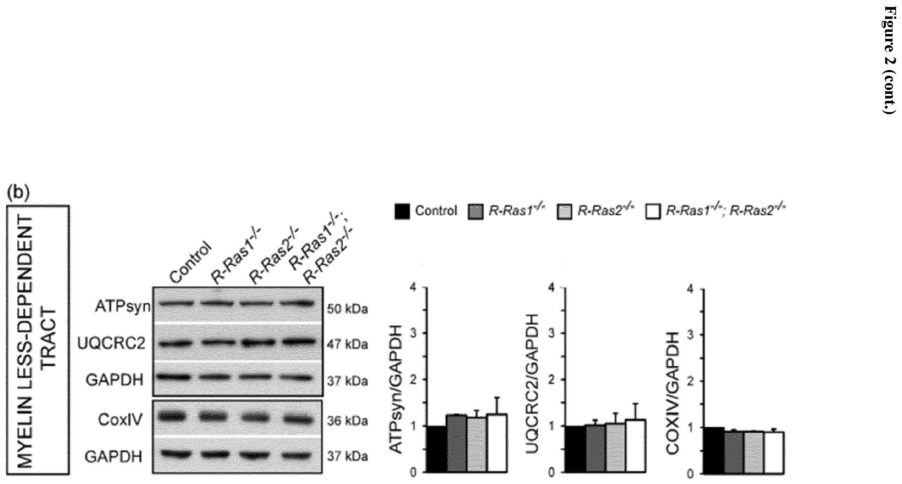

**Figure 2****.** Levels of mitochondrial complexes III, IV, and V are increased in the absence of R-Ras1. (a) Western blot analysis of ATP synthase (complex V), UQCRC2 (complex III) and COXIV (complex IV) in the optic nerve lysates of adult mice (P90) show an increment in the protein levels of these mitochondrial complexes in *R-Ras1*^{*-*/*-*} single and double-mutants compared to controls, in myelin-dependent tracts. Quantification of western blot evidenced significant increase in mitochondrial complexes III, IV, and V in *R-Ras1*^{*-*/*-*} single and double-mutant mice compared to controls. ATP synthase showed significant differences in *R-Ras1*^{*-*/*-*}(***p<0.001) and the double-mutant mice (*p<0.05). UQCRC2 showed significant differences in the double-mutant mice (***p<0.001) compared to the controls. COXIV showed significant differences in *R-Ras1*^{*-*/*-*} (***p<0.001) and the double-mutant mice *(***p<0.001).* (b) Western blot analysis of ATP synthase (complex V), UQCRC2 (complex III), and COXIV (complex IV) in myelin less-dependent tracts lysates of the adult mice (P90) shows no differences in the levels of these mitochondrial complexes in myelin less-dependent tracts. Quantification of western blot did not show any difference in mitochondrial complex III (UQCRC2), IV (COXIV), and V (ATP synthase) in single and double-mutant mice compared to controls (n = 4 mice per genotype). Bar graph represents mean ± SD of the change, relative to that of controls, in measurements normalized to GAPDH. Two-tailed Student t-test was used for the statistical analysis. These experiments were performed three times. GADPH, glyceraldehyde 3-phosphate dehydrogenase; SD, standard deviation.

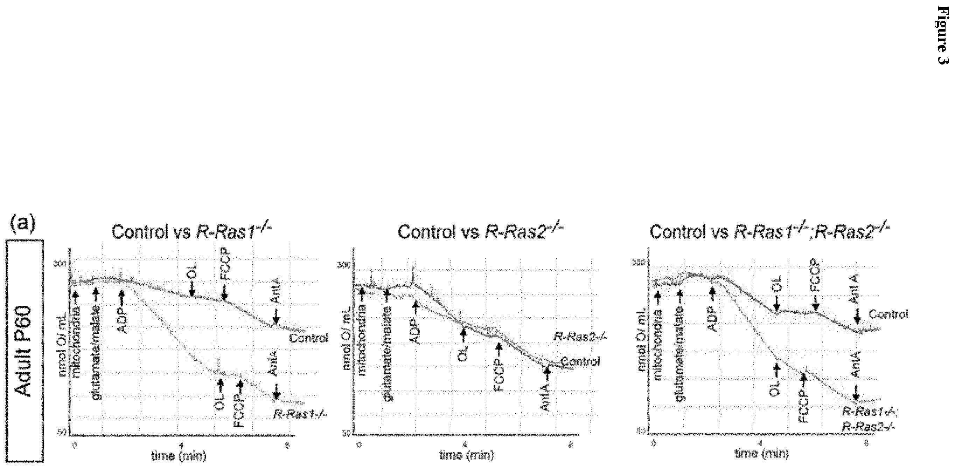

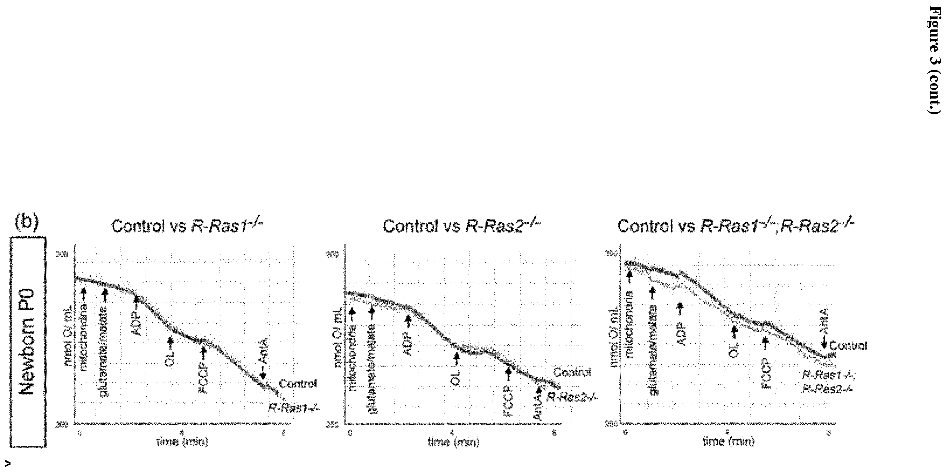

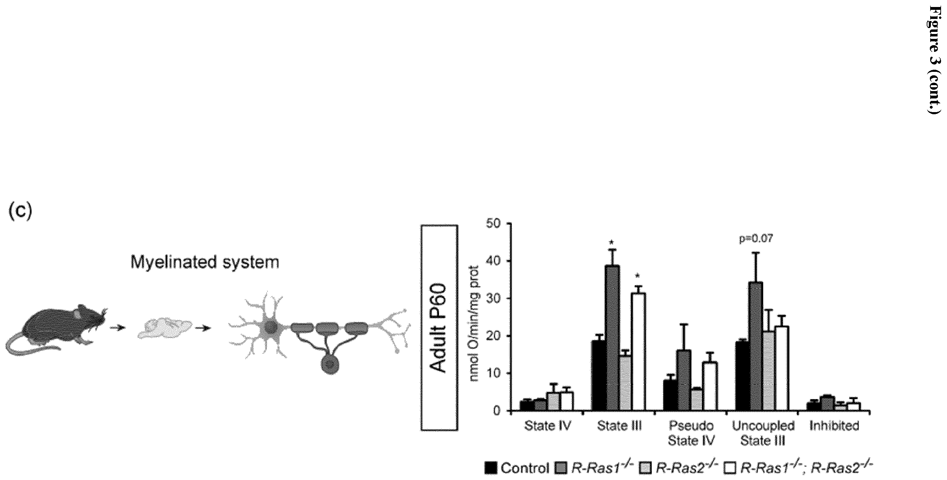

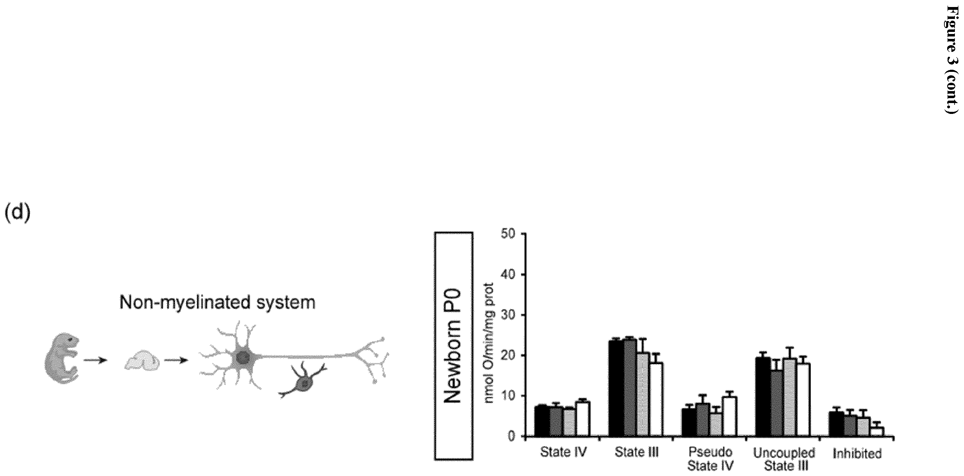

**Figure 3****.** Myelin deficiency in *R-Ras1*^{*-*/*-*} mice leads to an increase in mitochondrial activity. (a) Polarographic profiles of isolated mitochondria from the brain extracts of controls and mutant adult mice (P90) in the presence of 10 nM glutamate/malate, 0.5 mM ADP, 5 µM oligomycin (O), 5 µM FCCP, and 1 µM antimycin A (AntA), show evidence of higher mitochondrial activity in *R-Ras1*^{*-*/*-*} single and double-mutants. (b) Polarographic profiles of isolated mitochondria from the brain extracts of controls and mutant newborn (P0) mice in the presence of 10 nM glutamate/malate, 0.5 mM ADP, 5 µM oligomycin (O), 5 µM FCCP, and 1 µM AntA, did not show any difference between the mitochondrial activity in single and double-mutants compared to controls. (c) Representative scheme of a myelinated system and graph showing the quantification of Clark-type electrode results in adult (P60) mice mitochondria. State III respiration is expressed in nmol O/min/mg protein. These data show significant differences between *R-Ras1*^{*-*/*-*} (*p<0.05) and the double-mutant mice (**p*<0.05). (d) Representative scheme of a non-myelinated system and graph showing the quantification of Clark-type electrode results in newborn (P0) mice brain mitochondria. State III respiration is expressed in nmol O/min/mg protein. This data did not show any significant differences (n = 4 mice per genotype). Two-tailed Student t-test was used for the statistical analysis.

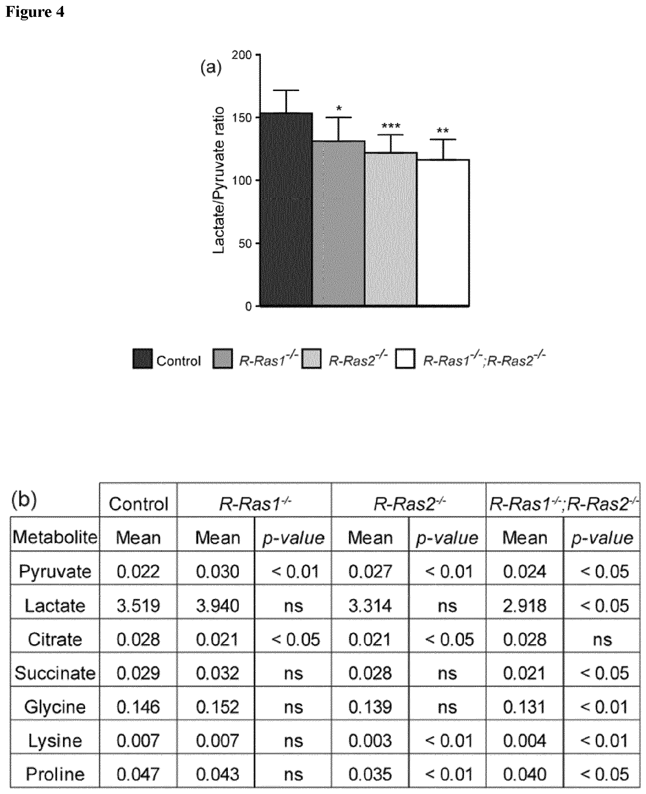

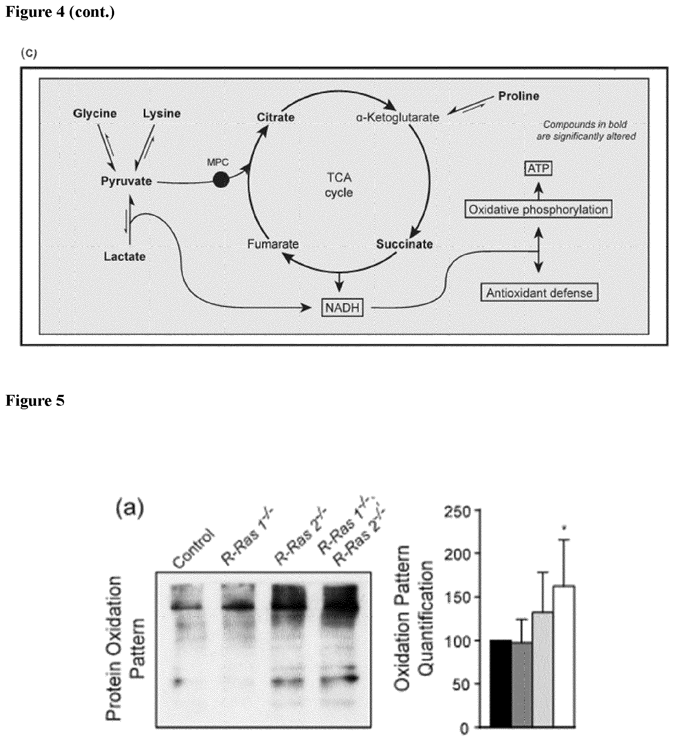

**Figure 4****.** Absence of R-Ras1 and/or R-Ras2 modifies the metabolism in the optic nerve. (a) Lactate/pyruvate ratio is decreased in single and double mutant mice compared to controls. Bar graph represents the mean ± SD and shows significant differences in *R-Ras1*^{*-*/*-*} (**p*<0.05), *R-Ras2*^{*-*/*-*} (***p<0.001), and *R-Ras1*^{*-*/}*⁻;R-Ras2*^{*-*/*-*} (**p<0.01) compared to controls. (b) Table showing the mean area under the curve of metabolites that are significantly altered in R-Ras single and double mutants compared to controls. Optic nerve samples were obtained from adult mice (P90) *(n* = 9 mice per genotype). For the metabolomics statistical analysis, Benjamini and Hochberg and Mann-Whitney U tests were used. (c) Schematic representation showing the possible link between the detected altered metabolites and their fate in support of ATP production and antioxidant defense. MPC: Mitochondrial pyruvate carrier; SD, standard deviation.

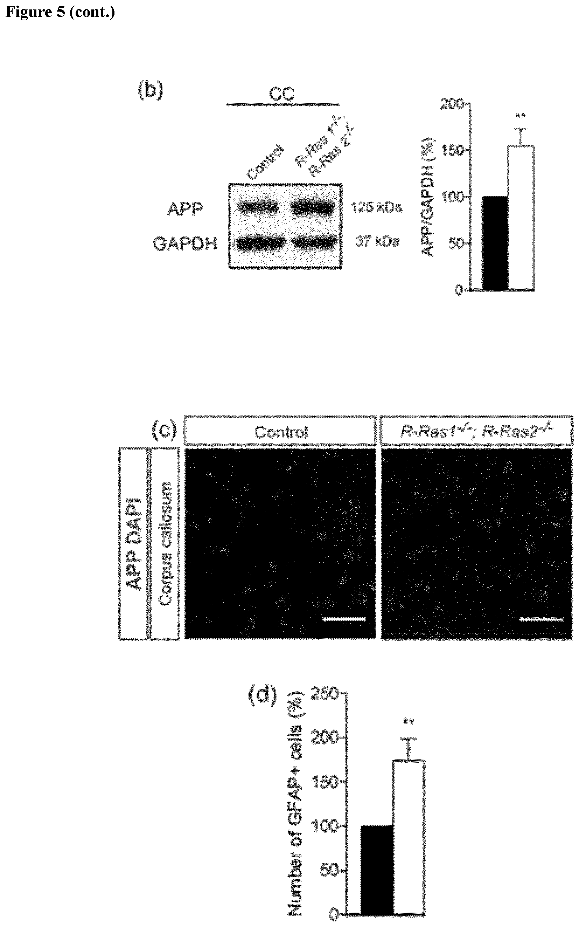

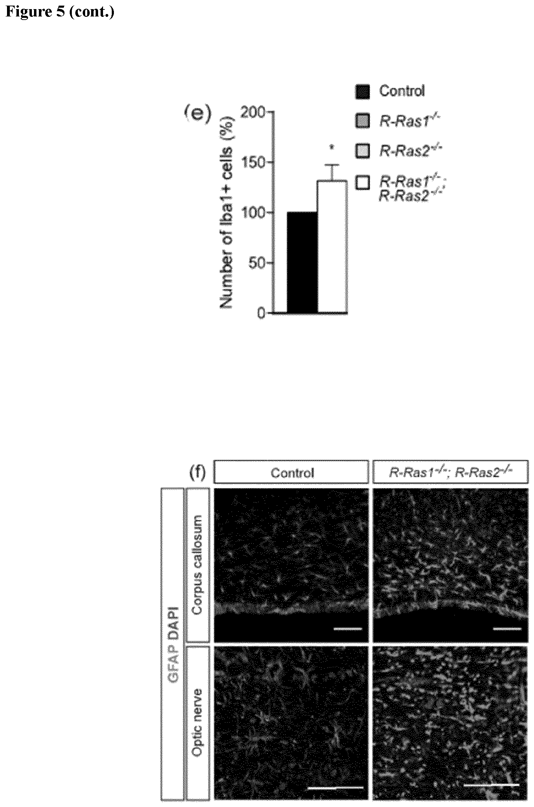

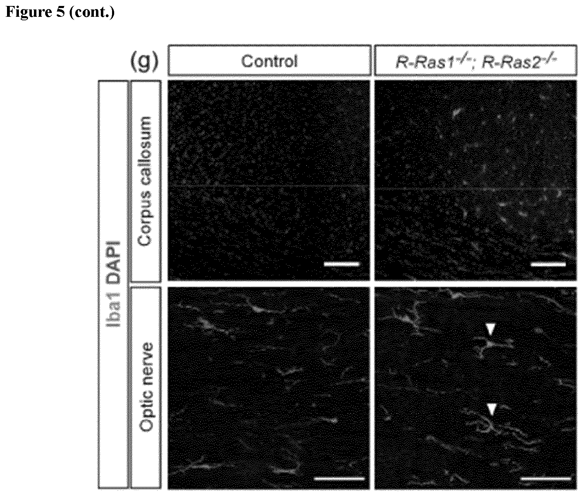

**Figure 5****.** Higher levels of protein oxidation accompanied by APP accumulation, astrogliosis, and microgliosis in *R-Ras1*^{*-*/}*⁻;R-Ras2*^{*-*/*-*} mutant mice. (a) Western blot of the protein oxidation pattern of optic nerve lysates from single and double R-Ras mutant adult mice (P90) compared to controls, showing an increase in protein oxidation pattern in the absence of R-Ras1 and R-Ras2 (*p<0.05). (b) Western blot in the cerebral cortex (CC) lysates of adult mice (P90) shows an increase in APP in *R-Ras1*^{*-*/}*⁻;R-Ras2*^{*-*/*-*} relative to controls (**p*<0.05). (c) Confocal microscopy images of the transverse sections of the CC of the adult (P90), immunostained against APP, revealed an increase in APP staining in *R-Ras1*^{*-*/}*⁻;R-Ras2*^{*-*/}*⁻.* Scale bar, 25 µm. (d) Graph of GFAP positive cells in the corpus callosum shows a significant increase in *R-Ras1*^{*-*/}*⁻;R-Ras2*^{*-*/*-*} mice relative to controls (**p<0.01). (e) Graph of Iba1 positive cells in the corpus callosum shows a significant increase in *R-Ras1*^{*-*/}*⁻;R-Ras2*^{*-*/*-*}mice relative to controls (*p<0.05). (f) Confocal microscopy images of the transverse sections of the Corpus callosum and the longitudinal sections of the optic nerve of adult mice (P90) immunostained against GFAP, revealed an increase in GFAP staining in *R-Ras1*^{*-*/}*⁻;R-Ras2*^{*-*/*-*} mice relative to controls. Scale bar, 50 µm. (g) Confocal microscopy images of the transverse sections of the corpus callosum and the longitudinal sections of optic nerve of adult mice (P90) immunostained with Iba1, revealed an increase in Iba1 staining in *R-Ras1*^{*-*/}*⁻;R-Ras2*^{*-*/*-*} mice relative to controls. Scale bar, 50 µm *(n* = 3 mice per genotype). Values in all bar graphs represent the mean ± SD of the change, relative to that of controls. Two-tailed Student t-test was used for the statistical analysis. SD, standard deviation; APP, amyloid precursor protein; GADPH, glyceraldehyde 3-phosphate dehydrogenase; GFAP, glial fibrillary acidic protein; Iba1, ionized calcium-binding adapter molecule 1

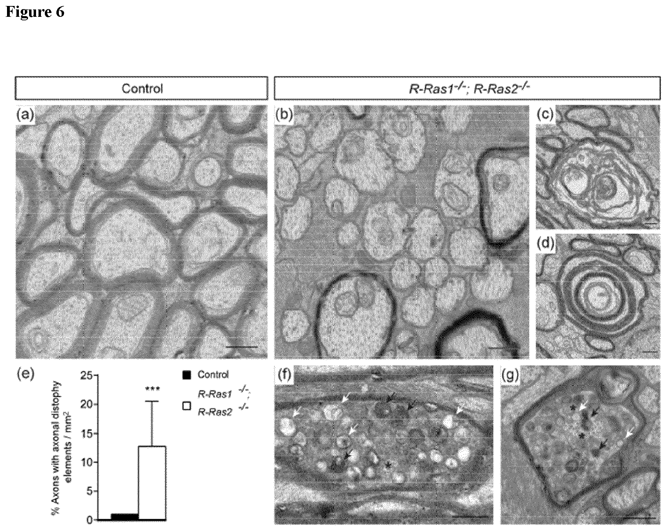

**Figure 6**. Transmission electron micrographs (TEMs) show astrogliosis and extensive axonal damage in the optic nerves of *R-Ras1*^{*-*/}*⁻;R-Ras2*^{*-*/*-*} mice. TEM of the optic nerves of controls (a) and *R-Ras1*^{*-*/}*⁻;R-Ras2*^{*-*/*-*} mice (b), astrogliosis is outlined in blue. *R-Ras1*^{*-*/}*⁻;R-Ras2*^{*-*/*-*} mice show more degenerating myelin sheaths presumptive of axonal degeneration (c, d). The R-*Ras1*^{*-*/}*⁻;R-Ras2*^{*-*/*-*} mice present with increased swollen axons (f and g) containing autophagosomes (white arrows), autophagolysosomes (black arrows), and multivesicular bodies (MVBs, black asterisks) relative to control mice. Axons with dystrophy elements were quantified. (e) Bar graph of axons with dystrophy elements/mm² showed increased MVBs and autophagy vesicles in the optic nerve of R- *R-Ras1*^{*-*/}*⁻;R-Ras2*^{*-*/*-*} mice compared to controls. Bar graph represents the mean ± SD (****p*<0.001). Two-tailed Student t-test was used for the statistical analysis. Scale bars: 500 nm. Images were taken at 12,000x times magnification (n = 3 mice per genotype). SD, standard deviation

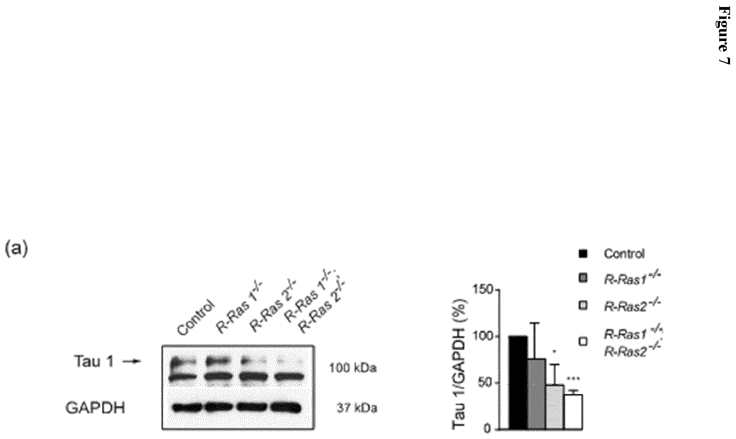

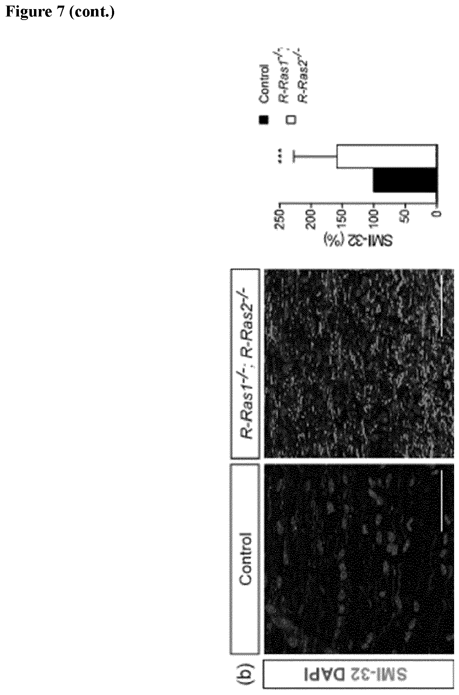

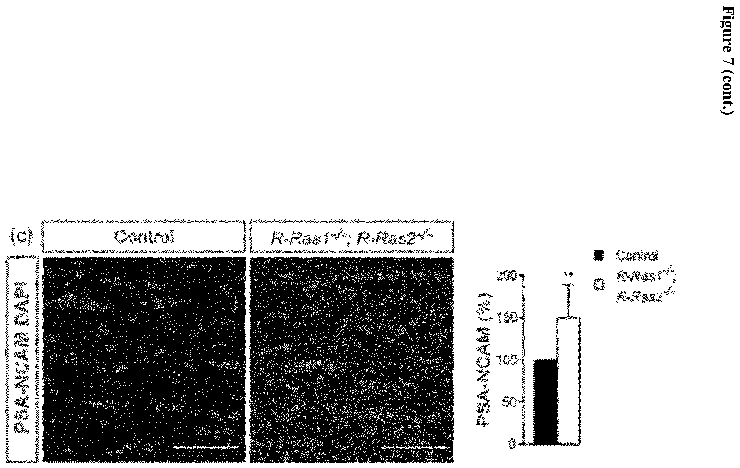

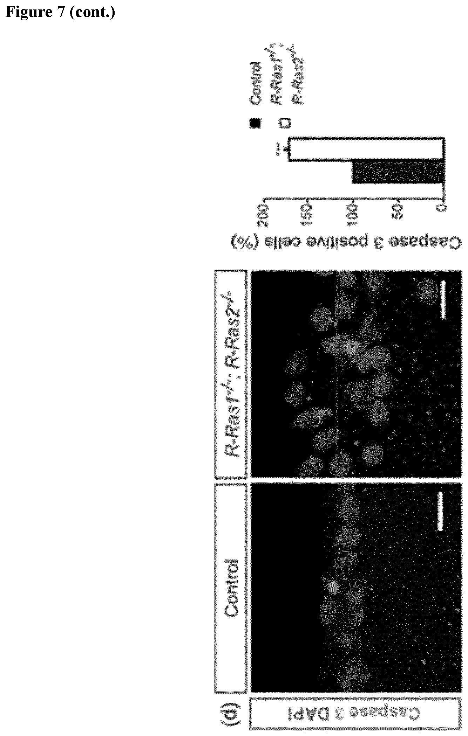

**Figure 7**. Absence of R-Ras1 and R-Ras2 causes the loss of integrity in the axonal cytoskeleton components associated with neuronal death. (a) Western blot of Tau 1 in the optic nerve lysates of adult mice (P90), showing a significant reduction in the Tau 1 phosphorylated isoform in *R-Ras2*^{*-*/*-*} and *R-Ras1*^{*-*/}*⁻;R-Ras2*^{*-*/*-*} mice compared with controls. These experiments were performed three times and results were normalized to GAPDH. Results show significant decrease in *R-Ras2*^{*-*/*-*} (**p*<0.05) and *R-Ras1*^{*-*/}*⁻;R-Ras2*^{*-*/*-*}(****p*<0.001). (b) Confocal microscopy images of the longitudinal sections of the optic nerves of adult mice (P90) immunostained with SMI-32 revealed an increase in staining in R-*Ras1*^{*-*/}*⁻;R-Ras2*^{*-*/*-*} mice relative to controls. Graph of SMI-32 mean fluorescence quantification showing a significant increase in double mutant mice compared to controls (***p<0.001). Scale bar, 50 µm. (c) Confocal microscopy images of the longitudinal sections of the optic nerves of adult mice (P90) immunostained with PSA-NCAM, revealed an increase in PSA-NCAM staining in *R-Ras1*^{*-*/}*⁻;R-Ras2*^{*-*/*-*} mice relative to controls. Graph of PSA-NCAM mean fluorescence quantification showing a significant increase in double mutant mice compared to controls (**p<0.01). Scale bar, 50 µm. (d) Confocal microscopy images of the transverse sections of the retina of adult mice (P90) immunostained with cleaved caspase 3, revealed an increase in caspase 3 positive cells in *R-Ras1*^{*-*/}*⁻;R-Ras2*^{*-*/*-*} mice relative to controls. Scale bar, 10 µm. Histological examination revealed an increase in apoptotic processes in the retinal ganglion cells (RGCs) in *R-Ras1*^{*-*/}*⁻;R-Ras2*^{*-*/*-*} (****p*<0.001). (*n* = 3 mice per genotype). Bar graph represents the mean ± SD of the change as percentage, relative to the controls measurements. Two-tailed Student t-test was used for the statistical analysis. PSA-NCAM, polysialylated-neural cell adhesion molecule; GADPH, glyceraldehyde 3-phosphate dehydrogenase; SD, standard deviation.

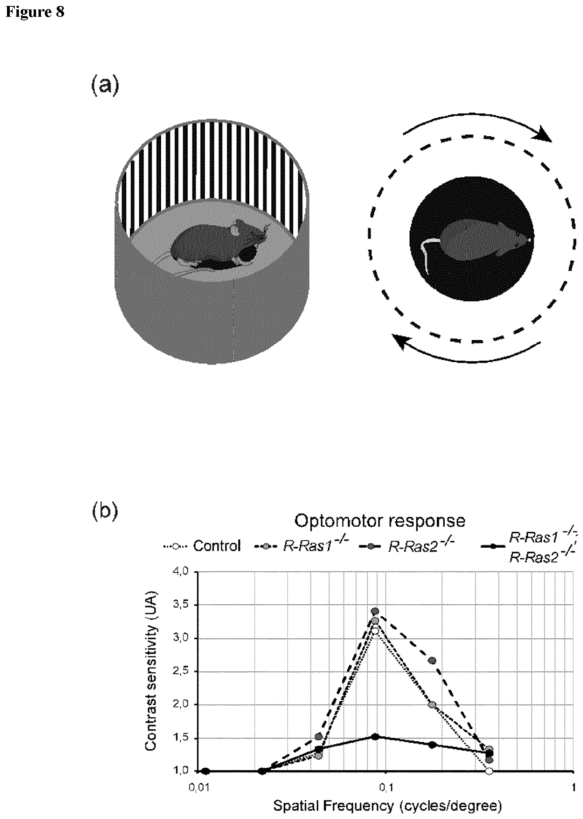

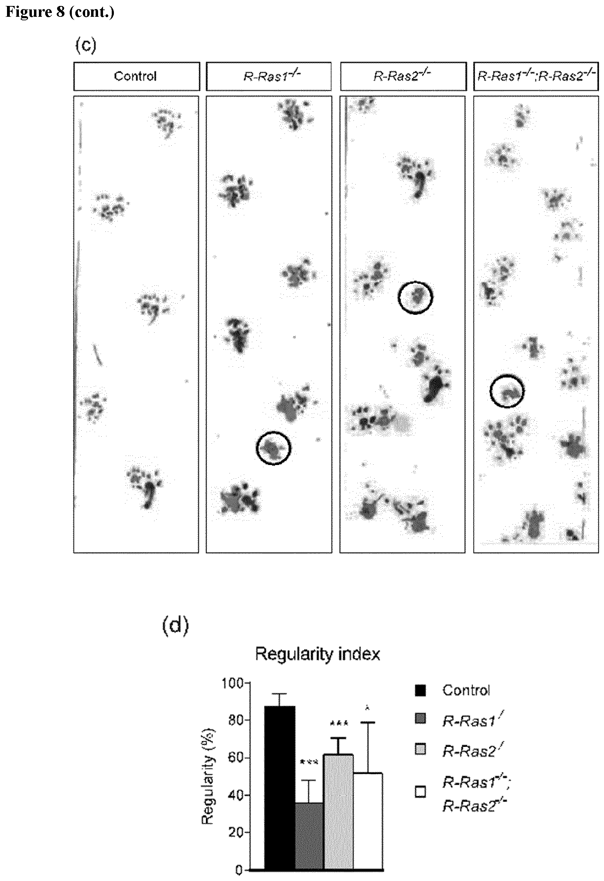

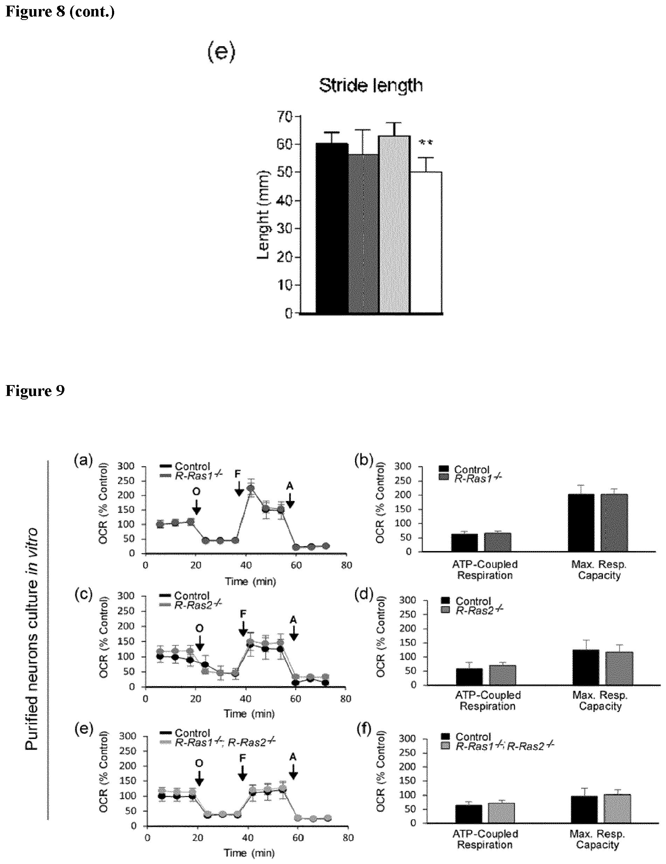

**Figure 8****.** Absence of R-Ras1 and R-Ras2 leads to loss of function. (a) Schematic representation of optomotor response test. (b) Graph of optokinetic responses of controls, R-*Ras1*^{*-*/}*⁻, R-Ras2*^{*-*/*-*} and *R-Ras1*^{*-*/}*⁻;R-Ras2*^{*-*/*-*} mice. Statistical differences between the double mutant and control mice (****p*<0.001) were found for intermediate spatial frequencies: 0.088, and 0.177 cycles/degree. No significant statistical differences were found between single mutant and control animals. Contrast sensitivity is represented as a function of spatial frequency as the mean ± SD. All experiments were performed in adult mice (P90) *(n* = 8 mice per genotype). ANOVA and Bonferroni post-test were used for statistical analysis. (c) Gait study reveals moderate motor impairment in *R-Ras1*^{*-*/}*⁻, R-Ras2,* and *R-Ras1*^{*-*/}*⁻;R-Ras2*^{*-*/*-*} mice. Images show forepaw prints in pink and hindpaw prints in green. Circles indicate missteps affecting mostly the forelimbs. (d) Gait analysis indicates low regularity index due to missteps in *R-Ras1*^{*-*/*-*} (****p*<0.001), *R-Ras2*^{*-*/*-*} (****p*<0.001), and *R-Ras1*^{*-*/}*⁻;R-Ras2*^{*-*/*-*}(**p*<0.05) compared to controls. (e) Stride length analysis reveals a decrease in stride length in *R-Ras1*^{*-*/}*⁻;R-Ras2*^{*-*/*-*} (***p*<0.01) mice compared to controls (*n* = 5 mice per genotype). Two-tailed Student t-test was used for the statistical analysis. SD, standard deviation

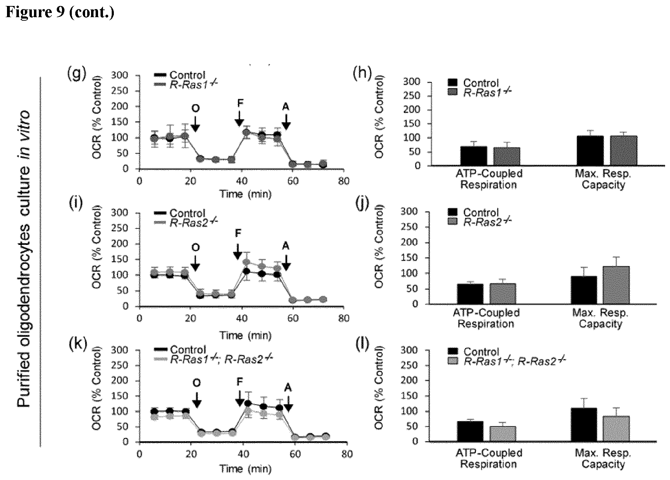

**Figure** 9. Purified oligodendrocytes and neurons from single and double mutant mice do not display intrinsic mitochondrial alterations. Seahorse assays developed on purified oligodendrocytes (OLs) and neurons of *R-Ras1*^{*-*/}*⁻, R-Ras2*^{*-*/*-*} and *R-Ras1*^{*-*/}*⁻;R-Ras2*^{*-*/*-*} did not show intrinsic mitochondrial alterations. (a-f) Measurements of oxygen consumption rate (OCR) as percentage of change relative to control, ATP-coupled respiration, and maximal respiratory capacity in neurons purified cultures *in vitro*, did not show any differences compared to controls (*n* = 3 independent cultures, with 6-12 wells per culture per genotype). (g-l) Measurements of OCR as percentage of change relative to control, ATP-coupled respiration, and maximal respiratory capacity in OLs purified cultures *in vitro,* did not show any differences compared to controls. Graph showing the results of OLs ATP-coupled respiration and maximal respiratory capacity, expressed in OCR as percentage of change relative to control (*n* = 3 independent cultures, with 6-12 wells per culture per genotype). Bar graph represents the mean ± SD of the change as percentage, relative to the control measurements. Two-tailed Student t-test was used for the statistical analysis.

### Detailed description of the invention

The present invention is illustrated by means of the Examples set below without the intention if limiting its scope of protection.

### Examples 1. Material and Methods.

### Example 1.1. Animals.

C57BL6 Mice were housed in specific pathogen-free conditions in a humidity- and temperature-controlled room, in a 12 h light/dark cycle, receiving water and food *ad libitum.* All experiments were performed in male and female mice. All animal procedures were approved by the corresponding institutional ethical committee (Centro de Biologia Molecular Severe Ochoa, CBMSO) and were performed in accordance with Spanish and European guidelines. All efforts were made to minimize animal suffering.

*R-Ras1*^{*-*/*-*} mice were generated at GenoWay (Lion, France) using the targeting construction BAL1-HR with a neomycin resistance cassette flanked by FRT sequences, inserted in intron 1 and LoxP sites, flanking exons 2 and 6. The construction was electroporated into embryonic stem cells derived from mouse 129Sv/Pas and selected by G418 antibiotic. Southern blot was used to verify the correct homologous recombination.

Heterozygous mice were crossed, and offspring littermates were genotyped by PCR (*R-Ras1*^{*-*/-} FW: 5'-SEQ ID NO: 1-3', *R-Ras1*^{*-*/*-*} RV: 5'- SEQ ID NO: 2-3', *R-Ras1*^{+/+} FW: 5'- SEQ ID NO: 3 -3 , *R-Ras1*^{+/+} RV: 5'- SEQ ID NO: 4 -3').

*R-Ras2*^{*-*/*-*} mice were generated at Lexicon Pharmaceuticals (Texas, USA) and were derived from embryonic stem cell clone OST361011 with insertion of retroviral VICTR37 in the middle of intron 1 of *R-Ras2.* Heterozygous mice were crossed and offspring littermates were genotyped by PCR (primer 1, 5'- SEQ ID NO: 5 -3'; primer 2, 5'- SEQ ID NO: 6-3'; primer 3, 5'- SEQ ID NO: 7 -3') (Delgado et al., 2009). *R-Ras* 2^{+/+} and *R-Ras2*^{-/-} transgenic mice were obtained by crossing heterozygous mice.

*R-Ras1*^{-/-} and *R-Ras2*^{-/-} mice were kindly provided by Prof. B. Alarcón (CBMSO, Spain). DKO *R-Ras1*^{*-*/}*⁻;R-Ras2*^{*-*/*-*} mice were generated by backcrossing individual lines *R-Ras1*^{*-*/*-*} and *R-Ras2*^{*-*/}*⁻.* Animals were maintained in a C57BL6J background. We used either male or female mice to perform the experiments. We defined the newborn as postnatal 0 (P0).

### Example 1.2. Western blotting.

Tissue samples (optic nerve, cerebral cortex, and the total brain of animals from P0 to adults [P90]) were dissected, resuspended in lysis buffer (50 mM Tris pH 8.0, 150 mM NaCl, 1% NP40, 2 mM EDTA, 0.1% SDS, 0.5% deoxycholate, and protease inhibition mixture; Roche 11697498001), and phenylmethane sulfonyl fluoride (PMSF). Lysates were denatured by boiling them for 5 min in protein loading buffer [50 mM Tris-HCl pH 6.8, 2% SDS, 10% glycerol, 1% β-mercaptoethanol (BME), 12.5 mM EDTA, and 0.02% bromophenol blue] and resolved in 10-12% SDSP-gels in the presence of BME. Gels were run at constant current starting at 90 or 100 V. After electrophoresis, samples were transferred by electroblotting onto a polyvinylidene difluoride (PVDF) membrane in a semidry electroblotting system (Transblot-turbo. Bio Rad laboratories; California, USA) at 1.2 mA/cm² for 35-40 min. Nonspecific protein binding was blocked by incubating the membrane with 5% non-fat milk in TBS-Tween-20 for 1 h at room temperature. Membranes were incubated overnight with the pertinent primary antibodies in the blocking buffer: mouse anti-APP 1:1000 (Millipore catalog#MAB348, RRID:AB_94882), mouse anti-ATP synthase (subunit *α*) 1:1000 (Invitrogen catalog# 459240), mouse anti-UQCRC2 (ubiquinol-cytochrome c reductase complex) 1:3000 (Thermo Fisher Scientific catalog# PA5-30204), and mouse anti-CoxIV (cytochrome oxidase subunit IV) 1:1000 (Molecular Probes catalog# A-21348). Other antibodies used were mouse anti-GAPDH (G9) 1:1000 (Santa Cruz Biotechnology catalog# sc-365062, RRID:AB_10847862), mouse anti-MBP (aa67-74) 1:200 (Bio-Rad / AbD Serotec catalog# MCA685S, RRID:AB_325009), and mouse anti-Tau 1 1:1000 that was kindly provided by Prof. J. Ávila (CBMSO, Madrid). After washing, blots were incubated for 1 h with appropriated peroxidase-conjugated secondary antibodies (Thermo Fisher Scientific). Labeled proteins were detected with the Chemiluminescence Reagent ECL (GE Healthcare).

### Example 1.3. Oxyblot^{®} (OxyBlot Protein Oxidation Detection Kit^{®}).

OxyBlot assay was performed according to the manufacturer's specifications (Sigma-Aldrich catalog# S7150). It consists of the detection of the carbonyl groups that are introduced into the amino acid side chain after oxidative modification of proteins previously derivatizated to 2, 4-dinitrophenyl-hydrazone (DNP-hydrazone). Then DNP-derivatized proteins were resolved in 12% SDSP-gel samples and transferred by electroblotting onto a PVDF membrane (previously activated in EtOH 95%) in a semidry electroblotting system (Transblot-turbo, Bio Rad laboratories) at 1.2 mA/cm² for 35-40 min. Nonspecific protein binding was blocked by incubating the membrane with 5% non-fat milk in TBS-Tween-20 for 1 h at room temperature. Then the membranes were exposed to a primary rabbit anti-DNPH protein antibody 1:100 for 1 h, and then to a secondary goat anti-rabbit IgG (HRP-conjugated) antibody 1:300 for 1 h at room temperature (both antibodies were diluted in TBS-Tween-20). After incubations, the membranes were washed gently with TBS-Tween-20. Finally, the detection was performed with Chemiluminescence Reagent ECL (GE Healthcare).

### Example 1.4. Immunohistochemistry.

Animals were anesthetized (ketamine/xylazine) and perfused transcardially with 0.1 M phosphate-buffered saline (PBS; pH 7.4) followed by 4% paraformaldehyde in PBS. Perfused tissues were removed and postfixed in 4% paraformaldehyde at 4°C overnight. Then they were cryoprotected in 30% sucrose in PBS and embedded and frozen in a 7.5% gelatin in 15% sucrose solution. Then they were sectioned on a cryostat to produce 20 µm cryosections on Superfrost Plus microscope slides (Thermo Fisher Scientific). Sections were blocked for 1 h at room temperature with 10% fetal bovine serum in PBS containing 0.5% Triton-X 100 (blocking solution) and then incubated overnight at 4°C with the primary antibodies: mouse anti-APP 1:500 (Millipore catalog#MAB348, RRID:AB_94882), rabbit anti-GFAP 1:1000 (Dako catalog# Z0334, RRID:AB_10013382), rabbit anti-Iba1 1:500 (Wako catalog# 019-19741, RRID:AB_839504), rabbit anti-cleaved caspase 3 (Asp 175) 1:500 (Cell Signaling catalog# 9661, RRID:AB_2341188), mouse anti-SMI-32 1:1000 (Biolegend catalog# 801701, RRID:AB_2564642), or mouse anti-polysialylated-neural cell adhesion molecule (PSA-NCAM) 1:50 (Miltenyi Biotec 130-117-394, RRID:AB_2727931) diluted in blocking solution. After 3 washes, fluorescent-tagged secondary antibodies (Alexa-488 or Alexa- 647) were applied for 1 h at room temperature, and sections were counterstained with DAPI (Sigma-Aldrich catalog# 32670) and mounted in Aqua-polymount mounting medium (Polyscience catalog# 18606).

### Example 1.5. Confocal microscopy.

Fluorescence images were obtained using a confocal multispectral Leica TCS SP5 system (Leica Microsystems) controlled by Las AF v 2.7 software (Leica). Image acquisition was performed sequentially using a 40x/1.4NA oil immersion objectives and appropriate fluorochrome excitation lines (405 nm, 488 nm, and 647 nm for DAPI, Alexa-488 and Alexa-647, respectively). Mean intensity was quantified using Fiji/ImageJ software.

### Example 1.6. Electron microscopy.

Mutant and littermate control mice were anesthetized as indicated above, intracardially perfused with 4% paraformaldehyde and 2.5% glutaraldehyde in 0.1M PBS and treated in the same fixative overnight. Optic nerves were then removed after several washes in PBS and the sections were postfixed with 1% osmium tetroxide in double-distillated water and 1% potassium ferrocyanide 1 h at 4°C. After 3 washes with double-distillated water, they were treated with 0.15% of tanic acid in 0.1 M PBS, pH 7.4, and block-stained with 2% uranyl acetate in distilled water for 1 h room temperature, in darkness. Sections were then washed 3 times with double-distilled water and dehydrated in an ascending series of ethanol dilutions of up to 100% at 4°C. The following infiltration was made with propylene oxide:EtOH (1: 1; v:v) for 5 min, propylene oxide 3 times at 15 min each, propylene oxide:Epon (1:1) (epoxy resin TAAB 812, TAAB laboratories) for 45 minutes, and 100% Epon 1 h and Epon 100% overnight. Encapsulation was made in flat molds where optic nerves were correctly orientated and then polymerized 48 h at 60°C. The optic nerve was cut in 70-80 nm sections on an ultramicrotome (Leica Ultracut UCT) with a diamond blade (Diatome) and collected on Cu-Pd boutonniere grids covered by Formvar. Staining of ultrathin sections was performed by drops of 2% aqueous uranyl acetate for 7 min, followed by Reynolds's lead citrate for 2 min. Ultrastructural analyses were performed with a JEM-1010 electron microscope (Jeol).

In cross sections, 12 photographs per mice were taken along the section covering the whole diameter of the optic nerve, using a CMOS 4K × 4K, F416 de TVIPS camera (Gauting). Images were taken at 12,000x times magnification. A total area of 302.58 µm² was accounted for per genotype. From these pictures, six in perfect condition that matched the mutants and controls, were used to identify astrogliosis processes.

In longitudinal sections, 10 photographs per mice were taken along the section covering the whole length of the optic nerve. For the mitochondrial quantification, images were taken at 6,000x times magnification and a total area of 2,017.2 µm² was accounted for per genotype. Mitochondria were identified as cylindrical double-membrane structures with interior crista membranes and classified between myelinated and non-myelinated axons. For the mitochondrial area, 394 axons in controls, 376 axons in *R-Ras1KO,* 469 axons in *R-Ras2KO,* and 498 axons in *DKO* were analyzed.

For the axonal dystrophy elements quantification, 10 photographs per genotype were analyzed in transversal sections of the optic nerve. Images were taken at 3,000x times magnification and a total area of 2,680 µm² was accounted for per genotype. Axonal dystrophy elements were identified as swollen axons containing autophagosomes, autophagolysosomes, and multivesicular bodies (MVB) structures in the axons.

Three animals per genotype were analyzed. Identical results were obtained independently, four times, by different investigators.

### Example 1.7. Brain mitochondrial isolation.

Fresh brains from the wild-type (WT), and P90 and P0 transgenic mice were minced and homogenized in a glass-glass homogenizer in buffer A (320 mM sucrose, 1 mM EDTA, and 10 mM Tris-HCl, and pH 7.4). Brain mitochondria were obtained by centrifugation. Briefly, unbroken cells and tissue were removed by centrifugation at 1,000 g for 5 min at 4°C. Synaptosomes present in the supernatant were permeabilized with 100 µM digitonine and mitochondria were obtained by supernatant centrifugation at 11,000 g for 15 min at 4°C. Mitochondria were resuspended in Buffer B (0.25 mM sucrose, 10 mM Tris-HCl, and ph 7.4, 0.1% (m/v) BSA).

### Example 1.8. Oxygen consumption rate (OCR).

Mitochondrial OCR in isolated mitochondria (200 µg protein) was determined with a Clark-type electrode. After proper calibration, mitochondria were placed in the chamber with continuous stirring in respiration buffer (225 mM sucrose, 5 mM MgCl2, 10 mM KCl, 10 mM phosphate buffer, 1 mM EGTA, 0.05% BSA and 10 mM Tris-HCl, pH 7.4). Four different compounds were added to test the mitochondrial function and describe changes in respiration states in mutant mice compared to controls. 10 µM glutamate plus malate were used as respiratory substrates for electron donation to mitochondrial complex I. The basal respiration state was measured prior to the addition of ADP (Pseudo state IV) and was controlled only by proton leak and substrate oxidation. Addition of 0.5 mM ADP under saturated substrate and oxygen (State III) allowed for the stimulation of the ATP synthase to test the oxidative phosphorylation (OXPHOS) activity. State III activity was ended by the addition of 5 µM oligomycin (O), an inhibitor of ATP synthase which made it possible to obtain State IV measurements. In this state, respiration was highly dependent on proton leak. By injection of 5 µM FCCP proton flux was restored, allowing for the measurements of the maximum respiratory capacity independently of ATP synthase activity (Uncoupled State III). Last, 1 µM antimycin A (Ant A) was used to inhibit all respiration and test the residual oxygen consumption.

### Example 1.9. Extracellular flux analysis ("Seahorse assay").

Seahorse assay on neurons and glial cells was performed as described previously (Katsu-Jiménez & Giménez-Cassina, 2019). OCR, ATP-coupled respiration and maximal respiration capacity were determined in real time using XF24 extracellular flux analyzer (Seahorse Bioscience-Agilent, Santa Clara CA). Purified and independent neurons and OLs cultures were seeded at 1.5×10⁵ or 4×10⁴ cells per well, respectively, in specific growth medium and then analyzed using the Seahorse XF24 extracellular flux analyzer. One hour prior to the experiment, culture medium was replaced by assay medium consisting of bicarbonate-free DMEM supplemented with 5 mM glucose and 1 mM sodium pyruvate. After the baseline measurement, the following sequential injections were made to determine ATP-coupled respiration and maximal respiratory capacity: 1 µM oligomycin, 0.5 µM carbonyl cyanide-4 (trifluoromethoxy) phenylhydrazone (FCCP), and 4 µM rotenone/antimycin A.

### Example 1.10. Metabolomics.

The metabolomics assay was performed at CEMBIO (Madrid, Spain), as described previously (Gonzalez-Riano et al., 2018). Briefly, the gas chromatography-mass spectrometry (GC-MS) analysis was developed with optic nerve samples from the controls, single, and double-mutant mice. Optic nerves were dissected from P90 mice after cervical dislocation. Samples were then kept at - 80°C immediately, until the day of the analysis. First, 100 µl of cold methanol (-20°C) containing 25 ppm of pentadecanoid acid used as an internal standard (IS) was added to each sample. Subsequently, samples were homogenized using a 2 mm particle size glass beads from TissueLyser LT homogenizer (Qiagen, Germany), centrifuged at 13.000 g at 4°C for 10 min, and 80 µl of the supernatant from each sample was transferred to the GC-MS vial. Supernatants were evaporated until dryness using a SpeedVac Concentrator System. Later, samples were chemically derivatized with two reactions, methoxymation and then silylation, for the GC-MS analysis. Before the analysis, 100 µL of heptane containing 10 ppm of methyl stearate C18:0 as a second IS was added. Finally, 2 µL of derivatized sample was injected in a split mode (1:10) into a GC instrument 7890A (Agilent Technologies, Germany) coupled to an inert mass spectrometer with the Triple-Axis detector 5975C (Agilent Technologies, Germany). The quality and the robustness of the analytical run was evaluated using quality control samples prepared by pooling equal volumes of homogenate from each sample. Raw data files were imported into Agilent Mass Hunter Unknown Analysis Tool 7.0 for the peak detection, deconvolution, and metabolite identification. The software executed a search against two targeted libraries to obtain a chemical identity for the compounds: Fiehn version 2013 and the CEMBIO internal spectrum library, always comparing both the retention time (RT) and the spectra extracted during deconvolution against each compound included in the library. A third commercial library-NIST (National Institute of Standards and Technology, library 2.2 version 2017) - was used for unknown identification. Those metabolites with a spectrum score of ≥ 80% and a concordant retention index based on the n-alkane scale were tentatively identified according to this library. Data were filtered by the signal variation coefficient (CV) in QC, considering acceptable values below 30%. Finally, altered metabolic pathways were analyzed using MetaboAnalyst^{®} 4.0 (Chong, Wishart, & Xia, 2019).

### Example 1.11. Optomotor response.

Optomotor test was performed as described previously (Prusky, Alam, Beekman, & Douglas, 2004). This test was proposed as a test of adequate animal behavior to evaluate the functional state of the retina's visual function in mice that suffer from retinal degenerations. Experimental animals respond to visualized rotating vertical bars by characteristic reflex movement of their head in the same direction to the bars' rotation. Contrast sensitivity is actually used as an evidence of visual discrimination (Umino, Solessio, & Barlow, 2008). A homemade optomotor device was built based on previous study (Prusky et al., 2004). Mice were placed at the center of a square array of computer monitors that displayed stimulus gratings. Mice were monitored using infrared television camera placed on top of the testing chamber. The visual test consisted of vertical black/white bars (gratings) of distinct spatial frequencies (0.011 to 0.355 cycles/degree) moving through the screens. Contrast sensitivity was calculated as the inverse of contrast threshold between white and black bars. Maximum contrast (value = 1) was measured for white screen luminance of 122,70 cd/m² and black screen luminance of 0.25 cd/m²). The Vision Egg tool was used for the light stimulation. The test started with the easiest stimulus for the mice, with a spatial frequency of 0.088 cycles/degree, a temporal frequency of 0.88 Hz, and a normalized contrast of 1.

### Example 1.12. Paw-print test.

This test allows for the detection of motor impairment (Maggipinto et al., 2017). Mice were habituated to the test room for 1 h before administration of the tests that were carried out during the light cycle. Mice paws in both forelimbs and hindlimbs were painted and the mice then walked on a 40 cm × 12 cm white piece of paper. Several gait parameters were measured using ImageJ software: stride length (the distance between two same-sided forelimbs or two same-sided hindlimbs), base width (side-to-side distance between the line of the two paws of fore or hindlimbs), paw placement (calculated as the angle between the long axes of the paw and the centerline of the mouse), and regularity index (percentage of correct step sequences over the total number of step cycle).

### Example 1.13. Statistical Analysis.

Quantitative data are shown as the mean ± standard deviation (SD). The experimental groups were compared using a two-tailed Student t-test. Statistical numeric data are provided in the figure legends. (*) means p<0.05; (**) means p<0.01; (***) means p<0.001. For metabolomics statistical analysis, we used Benjamini and Hochberg and Mann-Whitney U tests. An alpha level of 0.05 was considered significant. Outliers were identified using boxplots. These outliers were discarded as being outside the median trend. For the optomotor response statistical analysis, we used the two-way ANOVA test and Bonferroni post-tests.

### Example 2. Results.

### Example 2.1. Increased number of axonal mitochondria.

Here, we set out to investigate the metabolic adaptation and neuronal dysfunction resulting from the R-Ras loss of function, as mechanisms mimicking aspects of myelinating disorders. We first set out to perform additional electron microscopy analysis of optic nerve longitudinal sections to quantify the relationships between myelination defects, mitochondrial phenotype, and R-Ras genotypes. Consistent with our prior data, visual inspection of sections from *R-Ras1KO* mice showed thinner myelin layers whereas sections from *R-Ras2KO* mice had fewer myelinated axons relative to sections from WT mice. Consolidating these phenotypes, sections from *DKO* mice showed an overall more severe myelin phenotype **(****Figure** 1a). We found that axonal numbers of mitochondria were increased in *R-Ras2KO* and *DKO* mice. To determine whether there is a difference between increase in mitochondrial density and lack of axonal myelination, we next quantified the number of mitochondria present in myelinated and non-myelinated axons. We found that myelinated axons in mice across controls and all *R-Ras* genotypes contained roughly equal numbers of mitochondria (mitochondria per myelinated axon WT: 1.31 ± 0.15; *R-Ras1KO*: 1.26 ± 0.10; *R-Ras2KO:* 1.48 ± 0.36; and *DKO* mice: 2.41 ± 0.94) (**Figure 1b**). In contrast, axons lacking myelin sheath contained significantly more mitochondria in both *R-Ras2KO* and *DKO* mice (mitochondria per non-myelinated axons: 0.61 ± 0.18, *p*<0.05 and 0.75 ± 0.23, p<0.05) relative to WT and *R-Ras1KO* (mitochondria per non-myelinated axon: 0.22 ± 0.08 and 0.16 ± 0.08) (**Figure 1c**). This ratio revealed that a complete lack of myelin increases the number of mitochondria in *R-Ras2KO* and *DKO* mice whereas the normalized mitochondrial number count was comparable in WT and *R-Ras1KO* mice. To determinate whether the absence of R-Ras1 and/or R-Ras2 altered the mitochondrial size, we measured the mitochondrial area. Quantification revealed an increase in the average mitochondrial area in non-myelinated axons in *R-Ras1KO* mice (0.21 ± 0.06 µm² p<0.05), *R-Ras2KO* and *DKO* mice showed a decrease in average mitochondrial area in non-myelinated axons (for *R-Ras2KO:* 0.091 ± 0.05 µm² *p*<0.05 and for *DKO:* 0.083 ± 0.03 µm² p<0.001) relative to the controls (0.14 ± 0.08 µm²) (Figure 1e). No alterations in mitochondria size were observed in myelinated axons (Figure 1d). These data indicate that a lack of the myelin sheath leads to a mitochondrial adaptation, reflected as size alterations and an increase in the numbers of axonal mitochondria, whereas a mere decrease in the thickness of the myelin sheath surrounding the axons appears to be insufficient to impact on the number of the mitochondria (**Figure 1f**).

### Example 2.2. Increased protein levels of mitochondrial complexes.

We next asked whether R-Ras loss of function genotypes resulted in changes in mitochondrial activity. To answer this question, we quantified whether the levels of proteins executing mitochondrial OXPHOS in the optic nerve homogenates in adult mutant mice and WT controls. In OXPHOS, a group of complexes in the inner mitochondrial membrane create ATP by coordinating serial redox reactions to free up the energy captured in the double oxygen bond. With the western blot analysis, we found that abundance of complex III (UQCRC2; cytochrome c reductase), complex IV (COX IV; cytochrome c oxidase), and/or complex V (ATP synthase) subunits were increased in *R-Ras1KO* and *DKO* mice (Figure 2a). We did not detect any significant differences between *R-Ras2KO* homogenates and controls. The fold-change in protein level values for the different mitochondrial complexes relative to controls were as follows: ATP synthase: *R-Ras1KO* = 1.97 ± 0.09 (p<0.001); *R-Ras2KO* = 1.37 ± 0.76; *DKO* = 2.08 ± 0.84 (p<0.05). COX IV: *R-Ras1KO* = 2.45 ± 0.45 (p<0.001); *R-Ras2KO* = 1.13 ± 0.36; *DKO* = 2.67 ± 0.21 (p<0.001). UQCRC2: *R-Ras1KO* = 0.91 ± 0.29; *R-Ras2KO* = 0.93 ± 0.70; *DKO* = 3.40 ± 0.92 (p<0.001) (Figure 2a). To ascertain if the observed increases in protein levels of OXPHOS components were specific to myelin-dependent tracts, such as the optic nerve, we repeated the western blot analysis in homogenates of myelin less-dependent tracts. For ATP synthase, these were: *R-Ras1KO* = 1.22 ± 0.02 increase; *R-Ras2KO* = 1.18 ± 0.15; *DKO* = 1.24 ± 0.36. For COXIV: *R-Ras1KO* = 0.91 ± 0.04; *R-Ras2KO* = 0.91 ± 0.02; *DKO* = 0.87 ± 0.21. For UQCRC2: *R-Ras1KO* = 1.02 ± 0.07; *R-Ras2KO* = 1.05 ± 0.22; *DKO* = 1.13 ± 0.34 (**Figure 2b**). We found no differences in the protein levels of ATP synthase, UQCRC2, or COXIV in mutant mice in less-dependent myelin tissues (**Figure 2b**).

Taken together, these data show that the loss of R-Ras1 leads to increased levels of mitochondrial complexes (UQCRC2, COXIV, and ATP synthase) only, in myelin-dependent tracts, without causing alterations in weakly myelinated brain regions.

### Example 2.3. Increased oxygen consumption in the adult brain.

To understand whether R-Ras1 and R-Ras2 loss leads to functional changes in mitochondrial activity, we measured OCR in purified mitochondria, isolated from adult brains using a Clark-type electrode (**Figure 3a**). We did not observe quantifiable changes in State IV, Pseudo State IV, uncoupled-State III, and inhibited respiration across genotypes (**Figure 3a****,c**). However, we found that ADP-stimulated State III respiration (expressed as nmol O/ min / mg protein) was significantly elevated in *R-Ras1KO* (38.6 ± 7.4, p<0.05) and in *DKO* mitochondria (31.3 ± 3.1, p<0.05) relative to WT and *R-Ras2KO* (18.3 ± 3.4 and 14.6 ± 2.5) (**Figure 3c**).

We next asked whether the increase in OXPHOS activity in *R-Ras1KO* and *DKO* mice was dependent on the presence of myelin. To this end, we analyzed the OCR in a non-myelinated tissue (**Figure 3b****,d**), specifically, in mitochondria isolated from neonatal P0 mouse brain but we did not find any alterations in mitochondrial function in neonatal R-Ras mutant mice. State III respiration (nmol O / min / mg protein) was: 23.4 ± 1.4 for WT; 23.9 ± 1.0 for *R-Ras1KO;* 20.6 ± 6 for *R-Ras2KO;* and 18.8 ± 3.9 for *DKO* mice (**Figure 3d**). Taken together, these data show that myelin deficiency in adult mice resulting from the loss of R-Ras1 leads to a compensatory increase in mitochondrial activity, with no observed changes in mitochondrial respiration in non-myelin-dependent tissues.

### Example 2.4. Mitochondrial activity in OLs and isolated neurons.

To assess possible cell-intrinsic mitochondrial alterations in Ras mutant mice, we next compared mitochondrial activity of purified cultures of OLs and neurons isolated from R-Ras mutant and WT mice. To this end, we performed Seahorse assays to measure OCR in adherent cultures but did not detect any differences between mutant and control mice in either cell types (Figure 9 a,c,e) and OLs (Figure 9 g,i,k). Results of ATP-coupled respiration in cultured neurons: WT (62.4 ± 9.8 OCR) vs. *R-Ras1KO* (66.2 ± 7.2 OCR); WT (57.6 ± 23.8 OCR) vs. *R-Ras2KO* (69.9 ± 11.7 OCR); and WT (64.7 ± 13.1 OCR) vs. DKO (71.8 ± 10.3 OCR) (Figure 9 b,d,f). Results of maximal respiratory capacity in cultured neurons: WT (203.7 ± 32.1 OCR) vs. *R-Ras1KO* (203.4 ± 19.2 OCR); WT (125.2 ± 34.8 OCR) vs. R-*Ras2KO* (117.9 ± 25.5 OCR); and WT (96.3 ± 28.1 OCR) vs. DKO (102.1 ± 17.4 OCR) (Figure 9 b,d,f). In the case of ATP-coupled respiration in OLs: WT (69.7 ± 18.4 OCR) vs. *R-Ras1KO* (66.0 ± 19.5 OCR); WT (65.9 ± 7.1 OCR) vs. *R-Ras2KO (67.2* ± 13.2 OCR); and WT (66.5 ± 7.2 OCR) vs. DKO (50.9 ± 12.26 OCR) (Figure 9 h,j,l). Results of maximal respiratory capacity in OLs: WT (105.75 ± 21.4 OCR) vs. *R-Ras1KO* (106.4 ± 14.2 OCR); WT (91.7 ± 27.4 OCR) vs. *R-Ras2KO* (122.4 ± 30.6 OCR); and WT (110.0 ± 31.9 OCR) vs. DKO (82.6 ± 28.5 OCR) (Figure 9 h,j,l). These results proved the absence of intrinsic mitochondrial alterations within neurons and OLs independently, suggesting that R-Ras1 and R-Ras2 are essential, from a mitochondrial perspective, for correct energetic coupling between the OL and neuron.

### Example 2.5. Metabolism alterations in myelin-deficient tissues.

We next asked in what way the metabolic states of the axons are affected by an increase in number and/or activity of the mitochondria. In answer, we conducted an untargeted metabolomics-based study in the optic nerves of adult mice lacking R-Ras1 and/or R-Ras2, where we identified changes in the concentrations of multiple metabolites with GC-MS.

In R-Ras mutant mice, we found decreased lactate/pyruvate ratios in all *R-Ras* genotypes, but was most decreased in the DKO mice (WT: 153.53 ± 18.11; *R-Ras1KO*: 131.21 ± 18.91, p<0.05; *R-Ras2KO:* 121.75 ± 14.44, p<0.001; and *DKO:* 116.19 ± 16.42, p<0.01) (**Figure 4a**). Lower lactate/pyruvate ratio confirm increased ATP production in R-Ras mutant mice, which highlight increased mitochondrial function (Arnold & Rustin, 2001; Correia et al., 2006; Robinson, 2006).

We next used the web application MetaboAnalyst^{®}, commonly used to integrate metabolomic analysis (Chong et al., 2019), to ask how the changes we had observed in R-*Ras1* and/or *R-Ras2KO* mice affected different steps in the cellular metabolism. We found alterations for pyruvate (control: 0.022 ± 0.004; *R-Ras1KO*: 0.030 ± 0.007, p<0.01; *R-Ras2KO:* 0.027 ± 0.003, *p*<0.01; and *DKO*: 0.024 ± 0.003, p<0.05), lactate (control: 3.519 ± 0.640; *R-Ras1KO*: 3.940 ± 0.699; *R-Ras2KO:* 3.314 ± 0.405; and *DKO:* 2.918 ± 0.720, p<0.05), citrate (control: 0.028 ± 0.010; *R-Ras1KO*: 0.021 ± 0.004, p<0.05; *R-Ras2KO:* 0.021 ± 0.004, p<0.05; and *DKO:* 0.028 ± 0.010), succinate (control: 0.029 ± 0.007; *R-Ras1KO*: 0.032 ± 0.007; *R-Ras2KO:* 0.028 ± 0.004; and *DKO:* 0.021 ± 0.007, p<0.05), glycine (control: 0.146 ± 0.031; *R-Ras1KO*: 0.152 ± 0.052; *R-Ras2KO:* 0.139 ± 0.057; and *DKO:* 0.131 ± 0.040, p<0.01), lysine (control: 0.007 ± 0.002; *R-Ras1KO*: 0.007 ± 0.002; *R-Ras2KO:* 0.003 ± 0.001, p<0.01; and *DKO:* 0.004 ± 0.001, p<0.01), and proline (control: 0.047 ± 0.012; *R-Ras1KO*: 0.043 ± 0.013; *R-Ras2KO:* 0.035 ± 0.011, p<0.01; and *DKO:* 0.040 ± 0.013, *p*<0.05 ) (**Figure 4b**). Taken together, these data show that several metabolites directly involved in the Krebs cycle metabolism and amino acids are altered in R-Ras mutant mice (**Figure 4c**) in a way that suggest that mitochondria produce more energy relative to controls.

### Example 2.6. Degeneration and loss of axonal function.

We next sought to determine whether the increased mitochondrial activity we had observed led to an increase in ROS. To this end, we used a commercially available kit to detect protein oxidation levels in optic nerve homogenates from adult mutant mice compared to controls (Figure 5a). Although we did not observe significant differences in the single mutants relative to the WT, we detected the presence of a significantly elevated oxidizing environment in adult *DKO* mice (*R-Ras1KO*: 97.7 ± 26.5%; *R-Ras2KO:* 131.8 ± 46.6% and *DKO:* 162.4 ± 53.4%, *p*<0.05) (**Figure 5a**).

To know whether the increases in protein oxidation levels generated axonal pathology, we used immunohistochemical staining of the cerebral cortex sections against the amyloid precursor protein (APP). The extent of APP protein levels appeared to be correlated with histopathological lesion and thus suggests that APP detection serves as a sensitive marker in myelin diseases (Gehrmann, Banati, Cuzner, Kreutzberg, & Newcombe, 1995). *DKO* mice showed more intense staining compared to controls (**Figure 5c**). These findings were corroborated by western blot analysis in cerebral cortex homogenates, where we found significantly increased APP in *DKO* mice relative to WT (*DKO* 154.52 ± 18.71%, p<0.01) (**Figure 5b**).

To know whether axonal damage was accompanied by astrogliosis or/and microgliosis, we used immunostaining with glial fibrillary acidic protein (GFAP) and ionized calcium-binding adapter molecule 1 (Iba1) in the optic nerve and corpus callosum sections (**Figure 5f****,g**). *DKO* mice showed an increase of 173.95 ± 24.71% relative to control (p<0.01) in GFAP positive cells (**Figure 5d**). GFAP positive astrocytes from *DKO* mice exhibited morphologic changes characteristic of pathological conditions such as soma and processes hypertrophy (**Figure 5f**) (Escartin, Guillemaud, & Carrillo-de Sauvage, 2019). In addition, *DKO* mice showed an increase in Iba1 positive cells of 131.75 ± 16.08% relative to control (*p*<0.05) (**Figure 5e**). *DKO* microglia revealed an inflammatory-related morphology (**Figure 5g**, white arrow heads).

Furthermore, transmission electron micrographs (TEMs) confirmed axonal damage in the optic nerves of *DKO* mice relative to controls (**Figure 6a****-d,f,g**). *DKO* showed increased astrocytic processes compared to WT (**Figure 6a****,b**) (outlined in blue). In addition, *DKO* mice have more degenerating myelin sheaths, presumptive of axonal degeneration and increased swollen axons containing autophagosomes (**Figure 6f****,g**, white arrows), autophagolysosomes (**Figure 6f****,g**, black arrows), and multivesicular bodies (MVBs) (Figure 6f,g, black asterisks). Quantification of axonal damage elements showed an increase of 12.81±7.74 per mm² relative to controls (*p*<0.001) (**Figure 6e**). The data above corroborate extensive axonal pathology (Edgar et al., 2009; Ray et al., 2017).

We then asked whether the degree of phosphorylation/dephosphorylation of cytoskeletal elements would allow us to assess how axonal health was affected in R-Ras mutant mice. To this end, we performed western blot analysis in single and double mutant adult mice with antibodies for Tau 1, neurofilament heavy chain (NFH) and PSA-NCAM. In optic nerve homogenates, we found that the phosphorylated form of Tau 1 was significantly decreased in *R-Ras2KO* and *DKO* mice relative to WT (*R-Ras1KO*: 75.9 ± 38.5%; *R-Ras2KO:* 47.7 ± 22.1%, p<0.01; and *DKO* 37.4 ± 3.8%, *p*<0.001) (**Figure 7a**). In the longitudinal optic nerve sections, we identified the non-phosphorylated form of NFH by immunohistochemical staining with the specific SMI-32 antibody (**Figure 7b**) (Thangavel, Sahu, Van Hoesen, & Zaheer, 2009). Signal quantification revealed a significant increase in axonal dephosphorylation in *DKO* relative to WT mice (58.7 ± 25.5%, p<0.001) (**Figure 7b**). PSA-NCAM is commonly upregulated in myelin diseases (Charles, 2002); we next quantified PSA-NCAM levels by immunohistochemical staining in the longitudinal optic nerve sections. Similar to NFH, PSA-NCAM was significantly increased in *DKO* relative to WT mice (50.0 ± 12.9%, p<0.01) (**Figure 7c**).

To determine whether the axonal damage produced by the increase in protein oxidation compromised the neuronal survivability, we used immunohistochemical staining against active/cleaved caspase-3 in the retinal ganglion cells (RGCs). Histological examination revealed an increase in apoptotic processes in RGCs in *DKO* (169.02 ± 4.47%, p<0.001) relative to controls (**Figure 7d**).

Cumulatively, the increase in APP, GFAP, Iba1, and PSA-NCAM protein levels, and changes in the phosphorylation levels of the axonal cytoskeleton, together with the apoptotic processes, suggest the presence of axonal degeneration and neuronal loss in the double mutants.

To evaluate whether these alterations had functional consequences, we performed optokinetic reflex measurements, a test commonly used to evaluate retinal function in retinal degeneration mouse models, in controls and R-Ras mutant mice. In this assay, experimental animals were placed in an environment with a screen simulating rotating black and white vertical bars, and in which their characteristic head reflex movements in the same direction as the rotating bars were measured (**Figure 8a**). Contrast sensitivity was used to assess the degree of capacity for visual discrimination. We found that reflex responses induced by the bar rotation at different spatial frequencies were significantly lower in *DKO* mice relative to both single mutants and control animals (**Figure 8b**). Specifically, contrast sensitivity in the range of 0.011-0.355 cycles/degree was significantly weaker in *DKO* mice (p<0.001), indicating a strong visual impairment (**Figure 8b**).

To confirm how axonal degeneration affected other strongly myelin-dependent regions such as the spinal cord, we evaluated single and double mutant mice motor function by paw-print tests (Maggipinto et al., 2017). Single and double mutant mice showed modifications in the gait regularity, including new erratic footsteps (**Figure 8c**, circle paws). Percentage of correct step sequences over the total number of step cycles were: for control: 87.50 ± 6.66; *R-Ras1KO*: 35.87 ± 11.98 (*p*<0.001); *R-Ras2KO:* 61.67 ± 8.81 (*p*<0.001); and *DKO:* 51.66 ± 27.23 (*p*<0.05) (**Figure 8d**). In addition, *DKO* showed a decrease in stride length (control: 60.37 ± 3.93 mm and *DKO*: 50.30 ± 5.08 mm (*p*<0.05) (**Figure 8e**).

Taken together, these findings suggest that myelin deficiency caused by the loss of R-Ras1 and R-Ras2 leads to increased ROS levels, which in turn causes severe axonal degeneration and loss of function.

## Claims

1. *In vitro* method for obtaining a transgenic mouse model of myelin pathologies which comprises the disruption or inactivation of the gene R-Ras2, or R-Ras1 and R-Ras2, and determining and establishing the following parameters:
a. A 70% to 155% increase of immature oligodendrocytes in the double knock-out *R-Ras1*^{*-*/}*⁻R-Ras2*^{*-*/}*⁻* transgenic mouse model, with respect to the number of immature oligodendrocytes measured in healthy controls, determined by immunostaining with specific antibodies against Tcf4 and O4 and quantified by fluorescence microscopy;
b. At least 50% decrease of mature oligodendrocytes in the double knock-out R-*Ras1*^{*-*/}*⁻R-Ras2*^{*-*/}*⁻* transgenic mouse model, with respect to the number of mature oligodendrocytes measured in healthy controls, determined by immunostaining with specific antibodies against Olig2 and CC1 and quantified by fluorescence microscopy;
c. At least 20% of demyelinated axons in the double knock-out *R-Ras1*^{*-*/}*⁻R-Ras2*^{*-*/-} transgenic mouse, and at least 70% of demyelinated axons in the knock-out *R-Ras2*^{*-*/}*⁻* as compared with heathy controls, determined by electron microscopy;
d. Mitochondrial adaptations comprising:
i. Increased mitochondrial number per non-myelinated axon of 0.61 ± 0.18 for the knock-out *R-Ras2*^{*-*/}*⁻* and 0.75 ± 0.23 for the double knock-out *R-Ras1*^{*-*/}*⁻R-Ras2*^{*-*/}*⁻* as compared with the number of 0.22 ± 0.08 measured in healthy control, determined by electron microscopy;
ii. Decreased average mitochondrial area in non-myelinated axons of 0.091 ± 0.05 mm² for the knock-out *R-Ras2*^{*-*/}*⁻* and 0.083 ± 0.03 mm² for the double knock-out *R-Ras1*^{*-*/}*⁻R-Ras2*^{*-*/}*⁻* as compared with the number 0.14 ± 0.08 mm² measured in healthy control, determined by electron microscopy;
iii. Increased protein expression of the following respiratory chain elements in the double knock-out *R-Ras1*^{*-*/}*⁻R-Ras2*^{*-*/*-*}: ATPsyn fold change of 2.08 ± 0.84, COXIV fold change 2.67 ± 0.21 and UQCRC2 fold change 3.40 ± 0.92, relative to the expression measured in healthy control, determined by western-blot;
iv. ADP-stimulated State III in the double knock-out *R-Ras1*^{*-*/}*⁻R-Ras2*^{*-*/}*⁻* of 31.3 ± 3.1 nmol O/ min / mg protein relative to healthy control, determined with Clark electrode;
e. Metabolism alteration in myelin deficient tissues comprising:
i. Lactate/pyruvate ratios of 121.75 ± 14.44 for the knock-out R-Ras2^{-/-}and 116.19 ± 16.42 for the double knock-out *R-Ras1*^{*-*/}*⁻R-Ras2*^{*-*/}*⁻* as compared with the number 153.53 ± 18.11 measured in healthy control, determined by gas chromatography-mass spectrometry;
ii. Pyruvate amount of 0.027 ± 0.003 for the knock-out R-Ras2^{-/-} and 0.024 ± 0.003 for the double knock-out *R-Ras1*^{*-*/}*⁻R-Ras2*^{*-*/}*⁻* as compared with the number 0.022 ± 0.004 measured in healthy control, determined by gas chromatography-mass spectrometry;
iii.Lactate amount of 3.314 ± 0.405 for the knock-out R-Ras2^{-/-} and 2.918 ± 0.720 for the double knock-out *R-Ras1*^{*-*/}*⁻R-Ras2*^{*-*/}*⁻* as compared with the number 3.519 ± 0.640 measured in healthy control, determined by gas chromatography-mass spectrometry;
iv. Citrate amount of 0.021 ± 0.004 for the knock-out R-Ras2^{-/-} and 0.028 ± 0.010 for the double knock-out *R-Ras1*^{*-*/}*⁻R-Ras2*^{*-*/}*⁻* as compared with the number 0.028 ± 0.010 measured in healthy control, determined by gas chromatography-mass spectrometry;
v. Succinate amount of 0.028 ± 0.004for the knock-out R-Ras2^{-/-} and 0.021 ± 0.007 for the double knock-out *R-Ras1*^{*-*/}*⁻R-Ras2*^{*-*/}*⁻* as compared with the number 0.029 ± 0.007 measured in healthy control, determined by gas chromatography-mass spectrometry;
vi. Glycine amount of 0.139 ± 0.057 for the knock-out R-Ras2^{-/-} and 0.131 ± 0.040 for the double knock-out *R-Ras1*^{*-*/}*⁻R-Ras2*^{-/-} as compared with the number 0.146 ± 0.031 measured in healthy control, determined by gas chromatography-mass spectrometry;
vii. Lysine amount of 0.003 ± 0.001 for the knock-out R-Ras2^{-/-}and 0.004 ± 0.001 for the double knock-out *R-Ras1*^{*-*/}*⁻R-Ras2*^{*-*/}*⁻* as compared with the number 0.007 ± 0.002 measured in healthy control, determined by gas chromatography-mass spectrometry;
viii. Proline amount of 0.035 ± 0.011 for the knock-out R-Ras2^{-/-}and 0.040 ± 0.013 for the double knock-out *R-Ras1*^{*-*/}*⁻R-Ras2*^{*-*/}*⁻* as compared with the number 0.047 ± 0.012 measured in healthy control, determined by gas chromatography-mass spectrometry;
f. Axonal degeneration determined by the following parameters:
i. ROS increment for the double knock-out R-Ras1^{-/-}R-Ras2^{-/-} of 162.4 ± 53.4% compared to healthy control, determined by western-blot;
ii. APP increment for the double knock-out *R-Ras1*^{*-*/}*⁻R-Ras2*^{*-*/}*⁻* of 154.52 ± 18.71% relative to healthy control, determined by western-blot and confirmed by immunostaining and confocal microscopy;
iii. Astrogliosis for the double knock-out *R-Ras1*^{*-*/}*⁻R-Ras2*^{*-*/}*⁻* of 173.95 ± 24.71% relative to healthy control, determined by immunostaining with antibodies against GFAP and confocal microscopy;
iv. Microgliosis for the double knock-out *R-Ras1*^{*-*/}*⁻R-Ras2*^{*-*/}*⁻* of 131.75 ± 16.08% relative to healthy control, determined by immunostaining with antibodies against Iba1 and confocal microscopy;
v. Alteration of the phosphorylation state of the element of the axonal cytoskeleton Tau1: 47.7 ± 22.1% for the knock-out R-Ras2^{-/-} and 37.4 ± 3.8% for the double knock-out *R-Ras1*^{*-*/}*⁻R-Ras2*^{*-*/}*⁻* relative to healthy control, determined by western-blot;
vi. Alteration of the phosphorylation state of the element of the axonal cytoskeleton SMI-32: 58.7 ± 25.5% for the double knock-out *R-Ras1*^{*-*/}*⁻ R-Ras2*^{*-*/}*⁻* relative to healthy control, determined by immunostaining and confocal microscopy;
vii. Expression of PSA-NCAM 50.0 ± 12.9% for the double knock-out *R-Ras1*^{*-*/}*⁻R-Ras2*^{*-*/*-*}relative to healthy control, determined by immunostaining and confocal microscopy;
viii. Increase in apoptotic processes in ganglion cells of 169.02 ± 4.47% for the double knock-out R-Ras1^{-/-}R-Ras2^{-/-} relative to healthy control, determined by immunostaining and confocal microscopy;
g. Functional alterations comprising:
i. Percentage of correct step sequences over the total number of step cycles of 51.66 ± 27.23 for the double knock-out R-Ras1^{-/-}R-Ras2^{-/-}, 61.67 ± 8.81 for the knock-out R-Ras2^{-/-} as compared to the percentage determined in healthy control 87.50 ± 6.66, determined by paw-print test;
ii. Stride length of 50.30 ± 5.08 mm for the double knock-out R-Ras1^{-/-}R-Ras2^{-/-} as compared to the healthy control 60.37 ± 3.93 mm, determined by paw-print test;
iii. Contrast sensitivity in the range of 0.011-0.355 cycles/degree was significantly weaker for the double knock-out R-Ras1^{-/-}R-Ras2^{-/-}, determined by optomotor test.

2. A method for screening candidate compounds for treating myelin pathologies, which comprises: a) determining in the mouse model obtained according to claim 1 the level of expression of R-Ras1 and R-Ras2, and the parameters described in claim 1 a) to g) after administering the candidate molecule and b) where, if after administering the candidate molecule, the parameters described in claim 1 a) to g) are similar to the values determined in healthy control, this is indicative that the candidate molecule is effective in the treatment myelin pathologies.

3. Method, according to claim 2, wherein the myelin pathology is selected from the group comprising: neuromyelitis optica, multiple sclerosis, Charcot Marie Tooth disease and leukodystrophies.

## Patentansprüche

1. *In-vitro*-Verfahren zum Erhalten eines transgenen Mausmodells von Myelinpathologien, welches die Disruption oder die Inaktivierung des Gens R-Ras2, oder R-Ras1 und R-Ras2, und das Bestimmen und das Feststellen der folgenden Parameter umfasst:
a. eine 70%ige bis 155%ige Erhöhung von unreifen Oligodendrozyten im Doppel-Knockout-*R-Ras1*^{-/-}*R-Ras2*^{*-*/*-*}-transgenen Mausmodell, in Bezug auf die Anzahl von unreifen Oligodendrozyten gemessen in gesunden Kontrollen, bestimmt mittels Immunfärbung mit spezifischen Antikörpern gegen Tcf4 und O4 und quantifiziert mittels Fluoreszenzmikroskopie;
b. mindestens eine 50%ige Verringerung von reifen Oligodendrozyten im Doppel-Knockout-*R-Ras1*^{*-*/}*⁻R-Ras2*^{*-*/*-*}-transgenen Mausmodell, in Bezug auf die Anzahl von reifen Oligodendrozyten gemessen in gesunden Kontrollen, bestimmt mittels Immunfärbung mit spezifischen Antikörpern gegen Olig2 und CC1 und quantifiziert mittels Fluoreszenzmikroskopie;
c. mindestens 20% von demyelinisierten Axonen in der Doppel-Knockout-*R-Ras1*^{-/-}*R-Ras2*^{*-*/*-*} -transgenen Maus, und mindestens 70% von demyelinisierten Axonen im Knockout-R-*Ras2*^{*-*/}*⁻* im Vergleich zu gesunden Kontrollen, bestimmt mittels Elektronenmikroskopie;
d. mitochondriale Anpassungen umfassend:
i. eine erhöhte mitochondriale Anzahl pro nicht-myelinisiertes Axon von 0,61 ± 0,18 für das Knockout-*R-Ras2*^{-/-} und von 0,75 ± 0,23 für das Doppel-Knockout-R-*Ras1*^{*-*/}*⁻R-Ras2*^{*-*/}*⁻* im Vergleich zur Anzahl von 0,22 ± 0,08 gemessen in einer gesunden Kontrolle, bestimmt mittels Elektronenmikroskopie;
ii. einen verringerten durchschnittlichen mitochondrialen Bereich in nichtmyelinisierten Axonen von 0,091 ± 0,05 mm² für das Knockout-*R-Ras2*^{*-*/*-*} und von 0,083 ± 0,03 mm² für das Doppel-Knockout-*R-Ras1*^{*-*/}*⁻R-Ras2*^{-/-} im Vergleich zur Anzahl 0,14 ± 0,08 mm² gemessen in einer gesunden Kontrolle, bestimmt mittels Elektronenmikroskopie;
iii. eine erhöhte Proteinexpression der folgenden Atmungskettenelemente im Doppel-Knockout-*RRas1*^{-/-}*R-Ras2*^{-/-}: ATPsyn-Änderung von 2,08 ± 0,84, COXIV-Änderung von 2,67 ± 0,21 und UQCRC2-Änderung von 3,40 ± 0,92, bezüglich der Expression gemessen in einer gesunden Kontrolle, bestimmt mittels Western Blot;
iv. einen ADP-stimulierten Zustand III im Doppel-Knockout-*R-Ras1*^{*-*/}*⁻R-Ras2*^{-/-} von 31,3 ± 3,1 nmol O/min/mg Protein bezüglich einer gesunden Kontrolle, bestimmt mit einer Clark-Elektrode;
e. eine Stoffwechselveränderung in Myelin-defizienten Geweben umfassend:
i. Lactat/Pyruvat-Verhältnisse von 121,75 ± 14,44 für das Knockout-R-Ras2^{-/-} und von 116,19 ± 16,42 für das Doppel-Knockout-*R-Ras1*^{-/-}*R-Ras2*^{-/-} im Vergleich zur Anzahl 153,53 ± 18,11 gemessen in einer gesunden Kontrolle, bestimmt mittels Gaschromatographie-Massenspektrometrie;
ii. eine Pyruvatmenge von 0,027 ± 0,003 für das Knockout-R-Ras2^{-/-} und von 0,024 ± 0,003 für das Doppel-Knockout-*R-Ras1*^{-/-}*R-Ras2*^{-/-} im Vergleich zur Anzahl 0,022 ± 0,004 gemessen in einer gesunden Kontrolle, bestimmt mittels Gaschromatographie-Massenspektrometrie;
iii. eine Lactatmenge von 3,314 ± 0,405 für das Knockout-R-Ras2^{-/-} und von 2,918 ± 0,720 für das Doppel-Knockout-*R-Ras1*^{-/-}*R-Ras2*^{-/-} im Vergleich zur Anzahl 3,519 ± 0,640 gemessen in einer gesunden Kontrolle, bestimmt mittels Gaschromatographie-Massenspektrometrie;
iv. eine Citratmenge von 0,021 ± 0,004 für das Knockout-R-Ras2^{-/-} und von 0,028 ± 0,010 für das Doppel-Knockout-*R-Ras1*^{-/-}*R-Ras2*^{-/-} im Vergleich zur Anzahl 0,028 ± 0,010 gemessen in einer gesunden Kontrolle, bestimmt mittels Gaschromatographie-Massenspektrometrie;
v. eine Succinatmenge von 0,028 ± 0,004 für das Knockout-R-Ras2^{-/-} und von 0,021 ± 0,007 für das Doppel-Knockout-*R-Ras1*^{-/-}*R-Ras2*^{-/-} im Vergleich zur Anzahl 0,029 ± 0,007 gemessen in einer gesunden Kontrolle, bestimmt mittels Gaschromatographie-Massenspektrometrie;
vi. eine Glycinmenge von 0,139 ± 0,057 für das Knockout-R-Ras2^{-/-} und von 0,131 ± 0,040 für das Doppel-Knockout-*R-Ras1*^{-/-}*R-Ras2*^{-/-} im Vergleich zur Anzahl 0,146 ± 0,031 gemessen in einer gesunden Kontrolle, bestimmt mittels Gaschromatographie-Massenspektrometrie;
vii. eine Lysinmenge von 0,003 ± 0,001 für das Knockout-R-Ras2^{-/-} und von 0,004 ± 0,001 für das Doppel-Knockout-*R-Ras1*^{*-*/}*⁻R-Ras2*^{*-*/*-*} im Vergleich zur Anzahl 0,007 ± 0,002 gemessen in einer gesunden Kontrolle, bestimmt mittels Gaschromatographie-Massenspektrometrie;
viii. eine Prolinmenge von 0,035 ± 0,011 für das Knockout-R-Ras2^{-/-} und von 0,040 ± 0,013 für das Doppel-Knockout-*R-Ras1*^{-/-}*R-Ras2*^{-/-} im Vergleich zur Anzahl 0,047 ± 0,012 gemessen in einer gesunden Kontrolle, bestimmt mittels Gaschromatographie-Massenspektrometrie;
f. eine axonale Degeneration bestimmt mittels der folgenden Parameter:
i. eine ROS-Zunahme für das Doppel-Knockout-R-Ras1^{-/-}R-Ras2^{-/-} von 162,4 ± 53,4% im Vergleich zu einer gesunden Kontrolle, bestimmt mittels Western Blot;
ii. eine APP-Zunahme für das Doppel-Knockout-*R-Ras1*^{-/-}*R-Ras2*^{-/-} von 154,52 ± 18,71% bezüglich einer gesunden Kontrolle, bestimmt mittels Western Blot und bestätigt mittels Immunfärbung und konfokaler Mikroskopie;
iii. eine Astrogliose für das Doppel-Knockout-*R-Ras1*^{*-*/}*⁻R-Ras2*^{*-*/*-*} von 173,95 ± 24,71% bezüglich einer gesunden Kontrolle, bestimmt mittels Immunfärbung mit Antikörpern gegen GFAP und konfokaler Mikroskopie;
iv. eine Mikrogliose für das Doppel-Knockout-*R-Ras1*^{-/-}*R-Ras2*^{-/-} von 131,75 ± 16,08% bezüglich einer gesunden Kontrolle, bestimmt mittels Immunfärbung mit Antikörpern gegen Iba1 und konfokaler Mikroskopie;
v. eine Veränderung des Phosphorylierungszustands des Elements des axonalen Zytoskeletts Tau1: von 47,7 ± 22,1% für das Knockout-R-Ras2^{-/-} und von 37,4 ± 3,8% für das Doppel-Knockout-*R-Ras1*^{-/-}*R-Ras2*^{-/-} bezüglich einer gesunden Kontrolle, bestimmt mittels Western Blot;
vi. eine Veränderung des Phosphorylierungszustands des Elements des axonalen Zytoskeletts SMI-32: von 58,7 ± 25,5% für das Doppel-Knockout-*R-Ras1*^{-/-}*R-Ras2*^{-/-} bezüglich einer gesunden Kontrolle, bestimmt mittels Immunfärbung und konfokaler Mikroskopie;
vii. eine Expression von PSA-NCAM von 50,0 ± 12,9% für das Doppel-Knockout-R-*Ras1*^{*-*/}*⁻R-Ras2*^{*-*/*-*}bezüglich einer gesunden Kontrolle, bestimmt mittels Immunfärbung und konfokaler Mikroskopie;
viii. eine Erhöhung in apoptotischen Prozessen in Ganglienzellen von 169,02 ± 4,47% für das Doppel-Knockout-R-Ras1^{-/-}R-Ras2^{-/-} bezüglich einer gesunden Kontrolle, bestimmt mittels Immunfärbung und konfokaler Mikroskopie;
g. funktionelle Veränderungen umfassend:
i. einen Prozentsatz von richtigen Trittsequenzen zur gesamten Anzahl von Trittzyklen von 51,66 ± 27,23 für das Doppel-Knockout-R-Ras1^{-/-}R-Ras2^{-/-}, von 61,67 ± 8,81 für das Knockout-R-Ras2^{-/-} im Vergleich zum Prozentsatz bestimmt in einer gesunden Kontrolle von 87,50 ± 6,66, bestimmt mittels eines Pfotenabdrucktests;
ii. eine Schrittlänge von 50,30 ± 5,08 mm für das Doppel-Knockout-R-Ras1^{-/-}R-Ras2^{-/-} im Vergleich zur gesunden Kontrolle von 60,37 ± 3,93 mm, bestimmt mittels eines Pfotenabdrucktests;
iii. eine Kontrastempfindlichkeit im Bereich von 0,011-0,355 Zyklen/Grad war signifikant schwächer für das Doppel-Knockout-R-Ras1^{-/-}R-Ras2^{-/-}, bestimmt mittels eines optomotorischen Tests.

2. Verfahren zum Überprüfung von Kandidatenverbindungen zur Behandlung von Myelinpathologien, welches Folgendes umfasst: a) das Bestimmen im Mausmodell erhalten nach Anspruch 1 des Expressionsniveaus von R-Ras1 und R-Ras2, und der Parameter beschrieben in Anspruch 1 a) bis g) nach der Verabreichung des Kandidatenmoleküls und b) wobei, wenn nach der Verabreichung des Kandidatenmoleküls, die Parameter beschrieben in Anspruch 1 a) bis g) den in einer gesunden Kontrolle bestimmten Werten ähnlich sind, dies darauf schließen lässt, dass das Kandidatenmolekül bei der Behandlung von Myelinpathologien wirksam ist.

3. Verfahren nach Anspruch 2, wobei die Myelinpathologie aus der Gruppe umfassend Neuromyelitis optica, multiple Sklerose, Charcot-Marie-Tooth-Erkrankung und Leukodystrophien ausgewählt wird.

## Revendications

1. Procédé in vitro pour obtenir un modèle de souris transgénique de pathologies myéliniques qui comprend la disruption ou inactivation du gène R-Ras2, ou R-Ras1 et R-Ras2, et la détermination et l'établissement des paramètres suivants :
a. une augmentation de 70 % à 155 % d'oligodendrocytes immatures dans le modèle de souris transgénique double knock-out *R-Ras1*^{*-*/}*⁻R-Ras2*^{*-*/}*⁻* par rapport au nombre d'oligodendrocytes immatures mesuré chez des contrôles sains, déterminée par immunocoloration avec des anticorps spécifiques contre Tcf4 et O4 et quantifiée par microscopie à fluorescence ;
b. diminution d'au moins 50 % des oligodendrocytes matures dans le modèle de souris transgénique double knock-out *R-Ras1*^{*-*/}*⁻R-Ras2*^{*-*/*-*}, par rapport au nombre d'oligodendrocytes matures mesuré chez des contrôles sains, déterminée par immunocoloration avec des anticorps spécifiques contre Olig2 et CC1 et quantifiée par microscopie à fluorescence ;
c. au moins 20 % d'axones démyélinisés chez la souris transgénique double knock-out *R-Ras1*^{-/-}*R-Ras2*^{*-*/-}, et au moins 70 % d'axones démyélinisés chez le knock-out *R-Ras2*^{*-*/}*⁻* en comparaison avec des contrôles sains, déterminés par microscopie électronique ;
d. adaptations mitochondriales comprenant :
i. nombre de mitochondries augmenté par axone non myélinisé de 0,61 ± 0,18 pour le knock-out *R-Ras2*^{*-*/}*⁻* et de 0,75 ± 0,23 pour le double knock-out *R-Ras1*^{*-*/}*⁻R-Ras2*^{*-*/}*⁻* en comparaison avec le nombre de 0,22 ± 0,08 mesuré chez le contrôle sain, déterminé par microscopie électronique ;
ii. surface mitochondriale moyenne diminuée dans des axones non myélinisés de 0,091 ± 0,05 mm² pour le knock-out *R-Ras2*^{-/-} et de 0,083 ± 0,03 mm² pour le double knock-out R-*Ras1*^{*-*/}*⁻R-Ras2*^{*-*/}*⁻* en comparaison avec le nombre 0,14 ± 0,08 mm² mesuré chez le contrôle sain, déterminé par microscopie électronique ;
iii. expression de protéines augmentée des éléments de chaîne respiratoire suivants dans le double knock-out *R-Ras1*^{*-*/}*⁻R-Ras2*^{*-*/}*⁻* : valeur de changement de ATPsyn de 2,08 ± 0,84, valeur de changement de COXIV 2,67 ± 0,21 et valeur de changement de UQCRC2 de 3,40 ± 0,92, par rapport à l'expression mesurée chez le contrôle sain, déterminée par transfert Western ;
iv. état III stimulé par ADP dans le double knock-out *R-Ras1*^{*-*/}*⁻R-Ras2*^{*-*/}*⁻* de 31,3 ± 3,1 nmol O/ min / mg de protéine par rapport au contrôle sain, déterminé avec électrode de Clark ;
e. altération de métabolisme dans des tissus déficients en myéline comprenant :
i. rapports de lactate/pyruvate de 121,75 ± 14,44 pour le knock-out R-Ras2^{-/-} et de 116,19 ± 16,42 pour le double knock-out *R-Ras1*^{*-*/}*⁻R-Ras2*^{*-*/}*⁻* en comparaison avec le nombre 153,53 ± 18,11 mesuré chez le contrôle sain, déterminé par chromatographie gazeuse-spectrométrie de masse ;
ii. quantité de pyruvate de 0,027 ± 0,003 pour le knock-out R-Ras2^{-/-} et de 0,024 ± 0,003 pour le double knock-out *R-Ras1*^{*-*/}*⁻R-Ras2*^{*-*/}*⁻* en comparaison avec le nombre 0,022 ± 0,004 mesuré chez le contrôle sain, déterminé par chromatographie gazeuse-spectrométrie de masse ;
iii. quantité de lactate de 3,314 ± 0,405 pour le knock-out R-Ras2^{-/-} et de 2,918 ± 0,720 pour le double knock-out *R-Ras1*^{*-*/}*⁻R-Ras2*^{*-*/}*⁻* en comparaison avec le nombre 3,519 ± 0,640 mesuré chez le contrôle sain, déterminée par chromatographie gazeuse-spectrométrie de masse ;
iv. quantité de citrate de 0,021 ± 0,004 pour le knock-out R-Ras2^{-/-} et de 0,028 ± 0,010 pour le double knock-out *R-Ras1*^{*-*/}*⁻R-Ras2*^{*-*/}*⁻* en comparaison avec le nombre 0,028 ± 0,010 mesuré chez le contrôle sain, déterminé par chromatographie en phase gazeuse-spectrométrie de masse ;
v. quantité de succinate de 0,028 ± 0,004 pour le knock-out R-Ras2^{-/-} et de 0,021 ± 0,007 pour le double knock-out *R-Ras1*^{*-*/}*⁻R-Ras2*^{*-*/}*⁻* en comparaison avec le nombre 0,029 ± 0,007 mesuré chez le contrôle sain, déterminé par chromatographie en phase gazeuse-spectrométrie de masse ;
vi. quantité de glycine de 0,139 ± 0,057 pour le knock-out R-Ras2^{-/-} et de 0,131 ± 0,040 pour le double knock-out *R-Ras1*^{*-*/}*⁻R-Ras2*^{*-*/*-*}, en comparaison avec le nombre 0,146 ± 0,031 mesuré chez le contrôle sain, déterminé par chromatographie gazeuse-spectrométrie de masse ;
vii. quantité de lysine de 0,003 ± 0,001 pour le knock-out R-Ras2^{-/-} et de 0,004 ± 0,001 pour le double knock-out *R-Ras1*^{*-*/}*⁻R-Ras2*^{*-*/}*⁻* en comparaison avec le nombre 0,007 ± 0,002 mesuré chez le contrôle sain, déterminé par chromatographie gazeuse-spectrométrie de masse ;
viii. quantité de proline de 0,035 ± 0,011 pour le knock-out R-Ras2^{-/-} et de 0,040 ± 0,013 pour le double knock-out *R-Ras1*^{*-*/}*⁻R-Ras2*^{*-*/}*⁻* en comparaison avec le nombre 0,047 ± 0,012 mesurée chez le contrôle sain, déterminé par chromatographie gazeuse-spectrométrie de masse ;
f. dégénérescence axonale déterminée par les paramètres suivants :
i. augmentation de ROS pour le double knock-out R-Ras1^{-/-}R-Ras2^{-/-} de 162,4 ± 53,4 % comparé au contrôle sain, déterminée par transfert Western ;
ii. augmentation d'APP pour le double knock-out *R-Ras1*^{*-*/}*⁻R-Ras2*^{*-*/}*⁻* de 154,52 ± 18,71 % par rapport au contrôle sain, déterminée par transfert Western et confirmée par immunocoloration et microscopie confocale ;
iii. astrogliose pour le double knock-out *R-Ras1*^{*-*/}*⁻R-Ras2*^{*-*/}*⁻* de 173,95 ± 24,71 % par rapport au contrôle sain, déterminée par immunocoloration avec des anticorps contre la GFAP et microscopie confocale ;
iv. microgliose pour le double knock-out *R-Ras1*^{*-*/}*⁻R-Ras2*^{*-*/}*⁻* de 131,75 ± 16,08 % par rapport au contrôle sain, déterminée par immunocoloration avec des anticorps contre Iba1 et microscopie confocale ;
v. altération de l'état de phosphorylation de l'élément du cytosquelette axonal Tau 1 : 47,7 ± 22,1 % pour le knock-out R-Ras2^{-/-} et 37,4 ± 3,8 % pour le double knock-out *R-Ras1*^{-/-}*R-Ras2*^{*-*/}*⁻* par rapport au contrôle sain, déterminé par transfert Western ;
vi. altération de l'état de phosphorylation de l'élément du cytosquelette axonal SMI-32 : 58,7 ± 25,5 % pour le double knock-out *R-Ras1*^{*-*/}*⁻R-Ras2*^{*-*/}*⁻* par rapport au contrôle sain, déterminé par immunocoloration et microscopie confocale ;
vii. expression de PSA-NCAM de 50,0 ± 12,9 % pour le double knock-out *R-Ras1*^{*-*/}*⁻R-Ras2*^{*-*/*-*} par rapport au contrôle sain, déterminée par immunocoloration et microscopie confocale ;
viii. augmentation des processus apoptotiques dans des cellules de ganglion de 169,02 ± 4,47 % pour le double knock-out R-Ras1^{-/-}R-Ras2^{-/-} par rapport au contrôle sain, déterminée par immunocoloration et microscopie confocale ;
g. altérations fonctionnelles comprenant :
i. pourcentage de séquences d'étapes correctes sur le nombre total de cycles d'étapes de 51,66 ± 27,23 pour le double knock-out R-Ras1^{-/-}R-Ras2^{-/-}, 61,67 ± 8,81 pour le knock-out R-Ras2^{-/-} en comparaison avec le pourcentage déterminé chez le contrôle sain de 87,50 ± 6,66, déterminé par test d'empreinte de patte ;
ii. longueur de foulée de 50,30 ± 5,08 mm pour le double knock-out R-Ras1^{-/-}R-Ras2^{-/-} en comparaison avec le contrôle sain de 60,37 ± 3,93 mm, déterminée par test d'empreinte de patte ;
iii. la sensibilité au contraste dans la plage de 0,011-0,355 cycles/degré était significativement plus faible pour le double knock-out R-Ras1^{-/-}R-Ras2^{-/-}, déterminée par test optomoteur.

2. Procédé pour cribler des composés candidats pour traiter des pathologies myéliniques, qui comprend : a) déterminer dans le modèle de souris obtenu selon la revendication 1 le niveau d'expression de R-Ras1 et R-Ras2, et les paramètres décrits dans la revendication 1 a) à g) après avoir administré la molécule candidate et b) où, si après avoir administré la molécule candidate, les paramètres décrits dans la revendication 1 a) à g) sont similaires aux valeurs déterminées chez le contrôle sain, cela est révélateur que la molécule candidate est efficace dans le traitement de pathologies myéliniques.

3. Procédé, selon la revendication 2, dans lequel la pathologie myélinique est sélectionnée parmi le groupe comprenant : neuromyélite optique, sclérose multiple, maladie de Charcot-Marie-Tooth et leucodystrophies.
